# EUROPEAN PATENT APPLICATION

(11) **EP 2 354 162 A1**
(43) Date of publication of application: **10.08.2011**
(21) Application number: 10184453.8
(22) Date of filing: 12.09.2006
(51) Int. Cl.: C07K 16/28, A61K 39/395

(54) **Anti-CD3 antibody formulations**

(30) Priority: 12.09.2005 US 716311 P
(62) Divisional of application: 06790218.9
(71) Applicant: Novimmune SA, 1211 Genève (CH)
(72) Inventor: Elson, Greg, F-74160, Collonges sous Saleve (FR); Dean, Yann, F-74160 Bossey (FR); Kosco-Vilbois, Marie, F74270, Minzier (FR)
(74) Representative: Clarke, Victoria

(57) **Abstract**

This invention relates to therapeutic, diagnostic and/or prophylactic formulations and dosages of ani-CD3 antibodies, as well as to methods for using such formulations and dosages.

SEQUENCE LISTING

<110>  NovImmune SA, et al.

<120>  Anti-CD3 Antibody Formulations

<130>  23135-410-061

<150>  60/716,311
<151>  2005-09-12

<160>  74

<170>  PatentIn version 3.2

<210>  1
<211>  354
<212>  DNA
<213>  Homo sapiens

<400>  1
caggtgcagc tggtggagtc cgggggaggc gtggtccagc ctgggaggtc cctgagactc      60

tcctgtgcag cgtctggatt caagttcagt ggctatggca tgcactgggt ccgccaggct     120

ccaggcaagg ggctggagtg ggtggcagtt atatggtatg atggaagtaa gaaatactat     180

gtagactccg tgaagggccg cttcaccatc tccagagaca attccaagaa cacgctgtat     240

ctgcaaatga acagcctgag agccgaggac acggctgtgt attactgtgc gagacaaatg     300

ggctactggc acttcgatct ctggggccgt ggcaccctgg tcactgtctc ctca           354

<210>  2
<211>  118
<212>  PRT
<213>  Homo sapiens

<400>  2

<210>  3
<211>  324
<212>  DNA
<213>  Homo sapiens

<400>  3
gaaattgtgt tgacacagtc tccagccacc ctgtctttgt ctccagggga aagagccacc      60

ctctcctgca gggccagtca gagtgttagc agctacttag cctggtacca acagaaacct     120

ggccaggctc ccaggctcct catctatgat gcatccaaca gggccactgg catcccagcc     180

aggttcagtg gcagtgggtc tgggacagac ttcactctca ccatcagcag cctagagcct     240

gaagattttg cagtttatta ctgtcagcag cgtagcaact ggcctccgct cactttcggc     300

ggagggacca aggtggagat caaa                                            324

<210>  4
<211>  108
<212>  PRT
<213>  Homo sapiens

<400>  4

<210>  5
<211>  354
<212>  DNA
<213>  Homo sapiens

<400>  5
caggtgcagc tggtgcagtc cgggggaggc gtggtccagt ctgggaggtc cctgagactc      60

tcctgtgcag cgtctggatt caagttcagt ggctatggca tgcactgggt ccgccaggct     120

ccaggcaagg ggctggagtg ggtggcagtt atatggtatg atggaagtaa gaaatactat     180

gtagactccg tgaagggccg cttcaccatc tccagagaca attccaagaa cacgctgtat     240

ctgcaaatga acagcctgag aggcgaggac acggctgtgt attactgtgc gagacaaatg     300

ggctactggc acttcgatct ctggggccgt ggcaccctgg tcactgtctc ctca           354

<210>  6
<211>  118
<212>  PRT
<213>  Homo sapiens

<400>  6

Gln Val Gln Leu Val Gln Ser Gly Gly Gly Val Val Gln Ser Gly Arg
1               5                   10                  15

Ser Leu Arg Leu Ser Cys Ala Ala Ser Gly Phe Lys Phe Ser Gly Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Lys Lys Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Gly Glu Asp Thr Ala Val Tyr Tyr Cys
                85                  90                  95

Ala Arg Gln Met Gly Tyr Trp His Phe Asp Leu Trp Gly Arg Gly Thr
            100                 105                 110

Leu Val Thr Val Ser Ser
        115

<210>  7
<211>  324
<212>  DNA
<213>  Homo sapiens

<400>  7
gaaattgtgt tgacacagtc tccagccacc ctgtctttgt ctccagggga aagagccacc      60

ctctcctgca gggccagtca gagtgttagc agctacttag cctggtacca acagaaacct     120

ggccaggctc ccaggctcct catctatgat gcatccaaca gggccactgg catcccagcc     180

aggttcagtg gcagtgggtc tgggacagac ttcactctca ccatcagcag cctagagcct     240

gaagattttg cagtttatta ctgtcagcag cgtagcaact ggcctccgct cactttcggc     300

ggagggacca aggtggagat caaa                                            324

<210>  8
<211>  108
<212>  PRT
<213>  Homo sapiens

<400>  8

<210> 9
<211> 354
<212> DNA
<213> Homo sapiens

<400> 9
caggtgcagc tggtggagtc cgggggaggc gtggtccagc ctgggaggtc cctgagactc 60

tcctgtgcag cgtctggatt caccttcaga agctatggca tgcactgggt ccgccaggct 120

ccaggcaagg ggctggagtg ggtggcaatt atatggtatg atggaagtaa aaaaaactat 180

gcagactccg tgaagggccg attcaccatc tccagagaca attccaagaa cacgctgtat 240

ctgcaaatga acagcctgag agccgaggac acggctgtgt attactgtgc gagaggaact 300

gggtacaact ggttcgaccc ctggggccag ggaaccctgg tcaccgtctc ctca 354

<210> 10
<211> 118
<212> PRT
<213> Homo sapiens

<400> 10

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Arg Ser Tyr
            20                  25                  30

Gly Met His Trp Val Arg Gln Ala Pro Gly Lys Gly Leu Glu Trp Val
        35                  40                  45

Ala Ile Ile Trp Tyr Asp Gly Ser Lys Lys Asn Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Thr Gly Tyr Asn Trp Phe Asp Pro Trp Gly Gln Gly Thr
            100                 105                 110

Leu Val Thr Val Ser Ser
        115

<210>  11
<211>  324
<212>  DNA
<213>  Homo sapiens

<400>  11
gaaattgtgt tgacacagtc tccacgcacc ctgtctttgt ctccagggga aagagccacc      60

ctctcctgca gggccagtca gagtgttagc agcagctact tagcctggta ccagcagaaa     120

cctggccagg ctcccaggct cctcatctat ggtgcatcca gcagggccac tggcatccca     180

gacaggttca gtggcagtgg gtctgggaca gacttcactc tcaccatcag cagactggac     240

cctgaagatt ttgcagtgta ttactgtcag cagtatggta gctcaccgat caccttcggc     300

caagggacac gactggagat taaa                                            324

<210>  12
<211>  318
<212>  DNA
<213>  Homo sapiens

<400>  12
gacatcctga tgacccagtc tccatcctcc ctgtctgcat ctgtaggaga cagagtcacc      60

atcacttgcc gggcaagtca gggcattagc agtgctttag cctggtatca gcagaaacca     120

gggaaagctc ctaagctcct gatctattat gcatccagtt tgcaaagtgg ggtcccatca     180

aggttcagcg gcagtggatc tgggacggat tacactctca ccatcagcag cctgcagcct     240

gaagattttg caacttatta ctgtcaacag tattatagta ccctcacttt cggcggaggg     300

accaaggtgg agatcaaa                                                   318

<210>  13

<211>  318
<212>  DNA
<213>  Homo sapiens

<400>  13
gacatcgtga tgacccagtc tccatcctcc ctgtctgcat ctgtaggaga cagagtcacc      60

atcacttgcc gggcaagtca gggcattagc agtgctttag cctggtatca gcagaaacca     120

gggaaagctc ctaagctcct gatctatgat gcctccagtt tggaaagtgg ggtcccatca     180

aggttcagcg gcagtggatc tgggacagat ttcactctca ccatcagcag cctgcagcct     240

gaagattttg caacttatta ctgtcaacag tattatagta ccctcacttt cggcggaggg     300

accaaggtgg agatcaaa                                                   318

<210>  14
<211>  318
<212>  DNA
<213>  Homo sapiens

<400>  14
gacatccaga tgacccagtc tccattctcc ctgtctgcat ctgtaggaga cagagtcacc      60

atcacttgct gggccagtca gggcattagc agttatttag cctggtatca gcaaaaacca     120

gcaaaagccc ctaagctctt catctattat gcatccagtt tgcaaagtgg ggtcccatca     180

aggttcagcg gcagtggatc tgggacggat tacactctca ccatcagcag cctgcagcct     240

gaagattttg caacttatta ctgtcaacag tattatagta ccctcacttt cggcggaggg     300

accaaggtgg agatcaaa                                                   318

<210>  15
<211>  318
<212>  DNA
<213>  Homo sapiens

<400>  15
gacatcgaga tgacccagtc tccattctcc ctgtctgcat ctgtaggaga cagagtcacc      60

atcacttgct gggccagtca gggcattagc agttatttag cctggtatca gcaaaaacca     120

gcaaaagccc ctaagctctt catctattat gcatccagtt tgcaaagtgg ggtcccatca     180

aggttcagcg gcagtggatc tgggacggat tacactctca ccatcagcag cctgcagcct     240

gaagattttg caacttatta ctgtcaacag tattatagta ccctcacttt cggcggaggg     300

accaaggtgg agatcaaa                                                   318

<210>  16
<211>  108
<212>  PRT
<213>  Homo sapiens

<400>  16

Glu Ile Val Leu Thr Gln Ser Pro Arg Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Asp
65                  70                  75                  80

Pro Glu Asp Phe Ala Val Tyr Tyr Cys Gln Gln Tyr Gly Ser Ser Pro
                85                  90                  95

Ile Thr Phe Gly Gln Gly Thr Arg Leu Glu Ile Lys
            100                 105

<210>  17
<211>  106
<212>  PRT
<213>  Homo sapiens

<400>  17

Asp Ile Leu Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Tyr Ala Ser Ser Leu Gln Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Tyr Ser Thr Leu Thr
                85                  90                  95

Phe Gly Gly Gly Thr Lys Val Glu Ile Lys
            100                 105

<210>  18
<211>  106
<212>  PRT
<213>  Homo sapiens

<400>  18

Asp Ile Val Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Asp Ala Ser Ser Leu Gly Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Tyr Ser Thr Leu Thr
                85                  90                  95

Phe Gly Gly Gly Thr Lys Val Glu Ile Lys
            100                 105

<210>  19
<211>  106
<212>  PRT
<213>  Homo sapiens

<400>  19

Asp Ile Gln Met Thr Gln Ser Pro Phe Ser Leu Ser Ala Ser Val Gly
1               5                   10                  15

Asp Arg Val Thr Ile Thr Cys Trp Ala Ser Gln Gly Ile Ser Ser Tyr
            20                  25                  30

Leu Ala Trp Tyr Gln Gln Lys Pro Ala Lys Ala Pro Lys Leu Phe Ile
        35                  40                  45

Tyr Tyr Ala Ser Ser Leu Gln Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Tyr Ser Thr Leu Thr
                85                  90                  95

Phe Gly Gly Gly Thr Lys Val Glu Ile Lys
            100                 105

<210>  20
<211>  106
<212>  PRT
<213>  Homo sapiens

<400>  20

Asp Ile Glu Met Thr Gln Ser Pro Phe Ser Leu Ser Ala Ser Val Gly
1               5                   10                  15

Asp Arg Val Thr Ile Thr Cys Trp Ala Ser Gln Gly Ile Ser Ser Tyr
            20                  25                  30

Leu Ala Trp Tyr Gln Gln Lys Pro Ala Lys Ala Pro Lys Leu Phe Ile
        35                  40                  45

Tyr Tyr Ala Ser Ser Leu Gln Ser Gly Val Pro Ser Arg Phe Ser Gly
    50                  55                  60

Ser Gly Ser Gly Thr Asp Tyr Thr Leu Thr Ile Ser Ser Leu Gln Pro
65                  70                  75                  80

Glu Asp Phe Ala Thr Tyr Tyr Cys Gln Gln Tyr Tyr Ser Thr Leu Thr

85                    90                    95

Phe Gly Gly Gly Thr Lys Val Glu Ile Lys
            100                 105

<210>  21
<211>  354
<212>  DNA
<213>  Homo sapiens

<400>  21
caggtgcagc tggtgcagtc tgggggaggc gtggtccagc ctgggaggtc cctgagactc      60

tcctgtgtag cgtctggatt caccttcagt agctatggca tgcactgggt ccgccaggct     120

ccaggcaagg ggctggagtg ggtggcagct atatggtata atggaagaaa acaagactat     180

gcagactccg tgaagggccg attcaccatc tccagagaca attccaagaa cacgctgtat     240

ctgcaaatga acagcctgag agccgaggac acggctgtgt attactgtac gaggggaact     300

gggtacaatt ggttcgaccc ctggggccag ggaaccctgg tcaccgtctc ctca           354

<210>  22
<211>  118
<212>  PRT
<213>  Homo sapiens

<400>  22

Gln Val Gln Leu Val Gln Ser Gly Gly Gly Val Val Gln Pro Gly Arg
1               5                   10                  15

Ser Leu Arg Leu Ser Cys Val Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Ala Ile Trp Tyr Asn Gly Arg Lys Gln Asp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Thr Arg Gly Thr Gly Tyr Asn Trp Phe Asp Pro Trp Gly Gln Gly Thr
            100                 105                 110

Leu Val Thr Val Ser Ser
        115

<210>  23
<211>  321
<212>  DNA
<213>  Homo sapiens

<400>  23
gaaattgtgt tgacacagtc tccagccacc ctgtctttgt ctccagggga aagagccacc      60

ctctcctgca gggccagtca gagtgttagc agctacttag cctggtacca acagaaacct     120

ggccaggctc ccaggctcct catctatgat gcatccaaca gggccactgg catcccagcc     180

aggttcagtg gcagtgggtc tgggacagac ttcactctca ccatcagcag cctagagcct     240

gaagattttg cagtttatta ctgtcagcag cgtagcaact ggccgtggac gttcggccaa     300

gggaccaagg tggaaatcaa a                                               321

<210>  24
<211>  321
<212>  DNA
<213>  Homo sapiens

<400>  24
gccatccagt tgacccagtc tccatcctcc ctgtctgcat ctgtatgaga cagagtcacc      60

atcacttgcc gggcaagtca gggcattagc agtgctttag cctggtatca gcagaaacca     120

gggaaagctc ctaagctcct gatctatgat gcctccagtt tggaaagtgg ggtcccatca     180

aggttcagcg gcagtggatc tgggacagat ttcactctca ccatcagcag cctgcagcct     240

gaagattttg caacttatta ctgtcaacag tttaatagtt accctatcac cttcggccaa     300

gggacacgac tggagattaa a                                               321

<210>  25
<211>  107
<212>  PRT
<213>  Homo sapiens

<400>  25

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Trp
                85                  90                  95

Thr Phe Gly Gln Gly Thr Lys Val Glu Ile Lys
            100                 105

<210> 26
<211> 107
<212> PRT
<213> Homo sapiens

<400> 26

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro Ile
                85                  90                  95

Thr Phe Gly Gln Gly Thr Arg Leu Glu Ile Lys
            100                 105

<210>  27
<211>  5
<212>  PRT
<213>  Homo sapiens

<400>  27

Gly Tyr Gly Met His
1               5

<210>  28
<211>  17
<212>  PRT
<213>  Homo sapiens

<400>  28

Val Ile Trp Tyr Asp Gly Ser Lys Lys Tyr Tyr Val Asp Ser Val Lys
1               5                   10                  15

Gly

<210>  29
<211>  9
<212>  PRT
<213>  Homo sapiens

<400>  29

Gln Met Gly Tyr Trp His Phe Asp Leu
1               5

<210>  30
<211>  11
<212>  PRT
<213>  Homo sapiens

<400>  30

Arg Ala Ser Gln Ser Val Ser Ser Tyr Leu Ala
1               5                   10

<210>  31
<211>  7
<212>  PRT

<213>  Homo sapiens

<400>  31

Asp Ala Ser Asn Arg Ala Thr
1               5


<210>  32
<211>  10
<212>  PRT
<213>  Homo sapiens

<400>  32

Gln Gln Arg Ser Asn Trp Pro Pro Leu Thr
1               5                   10


<210>  33
<211>  5
<212>  PRT
<213>  Homo sapiens

<400>  33

Ser Tyr Gly Met His
1               5


<210>  34
<211>  17
<212>  PRT
<213>  Homo sapiens

<400>  34

Ile Ile Trp Tyr Asp Gly Ser Lys Lys Asn Tyr Ala Asp Ser Val Lys
1               5                   10                  15


Gly


<210>  35
<211>  9
<212>  PRT
<213>  Homo sapiens

<400>  35

Gly Thr Gly Tyr Asn Trp Phe Asp Pro
1               5

<210>  36
<211>  12
<212>  PRT
<213>  Homo sapiens

<400>  36

Arg Ala Ser Gln Ser Val Ser Ser Ser Tyr Leu Ala
1               5                   10


<210>  37
<211>  7
<212>  PRT
<213>  Homo sapiens

<400>  37

Gly Ala Ser Ser Arg Ala Thr
1               5


<210>  38
<211>  9
<212>  PRT
<213>  Homo sapiens

<400>  38

Gln Gln Tyr Gly Ser Ser Pro Ile Thr
1               5


<210>  39
<211>  11
<212>  PRT
<213>  Homo sapiens

<400>  39

Arg Ala Ser Gln Gly Ile Ser Ser Ala Leu Ala
1               5                   10


<210>  40
<211>  7
<212>  PRT
<213>  Homo sapiens

<400>  40

Tyr Ala Ser Ser Leu Gln Ser
1               5


<210>  41

<211>  8
<212>  PRT
<213>  Homo sapiens

<400>  41

Gln Gln Tyr Tyr Ser Thr Leu Thr
1               5


<210>  42
<211>  7
<212>  PRT
<213>  Homo sapiens

<400>  42

Asp Ala Ser Ser Leu Gly Ser
1               5


<210>  43
<211>  11
<212>  PRT
<213>  Homo sapiens

<400>  43

Trp Ala Ser Gln Gly Ile Ser Ser Tyr Leu Ala
1               5                   10


<210>  44
<211>  17
<212>  PRT
<213>  Homo sapiens

<400>  44

Ala Ile Trp Tyr Asn Gly Arg Lys Gln Asp Tyr Ala Asp Ser Val Lys
1               5                   10                  15


Gly


<210>  45
<211>  9
<212>  PRT
<213>  Homo sapiens

<400>  45

Gln Gln Arg Ser Asn Trp Pro Trp Thr
1               5

<210>  46
<211>  7
<212>  PRT
<213>  Homo sapiens

<400>  46

Asp Ala Ser Ser Leu Glu Ser
1               5


<210>  47
<211>  9
<212>  PRT
<213>  Homo sapiens

<400>  47

Gln Gln Phe Asn Ser Tyr Pro Ile Thr
1               5


<210>  48
<211>  98
<212>  PRT
<213>  Homo sapiens

<400>  48

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg

<210>  49
<211>  16
<212>  PRT
<213>  Homo sapiens

<400>  49

Asn Trp Phe Asp Pro Trp Gly Gln Gly Thr Leu Val Thr Val Ser Ser
1               5                   10                  15


<210>  50
<211>  17
<212>  PRT
<213>  Homo sapiens

<400>  50

Tyr Trp Tyr Phe Asp Leu Trp Gly Arg Gly Thr Leu Val Thr Val Ser
1               5                   10                  15


Ser


<210>  51
<211>  12
<212>  PRT
<213>  Homo sapiens

<400>  51

Leu Thr Phe Gly Gly Gly Thr Lys Val Glu Ile Lys
1               5                   10


<210>  52
<211>  12
<212>  PRT
<213>  Homo sapiens

<400>  52

Trp Thr Phe Gly Gln Gly Thr Lys Val Glu Ile Lys
1               5                   10


<210>  53
<211>  95
<212>  PRT
<213>  Homo sapiens

<400>  53

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Ala
            20                  25                  30

Leu Ala Trp Tyr Gln Gln Lys Pro Gly Lys Ala Pro Lys Leu Leu Ile
        35                  40                  45

Tyr Asp Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Phe Asn Ser Tyr Pro
                85                  90                  95

<210>  54
<211>  12
<212>  PRT
<213>  Homo sapiens

<400>  54

Ile Thr Phe Gly Gln Gly Thr Arg Leu Glu Ile Lys
1               5                   10

<210>  55
<211>  96
<212>  PRT
<213>  Homo sapiens

<400>  55

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

<210>  56
<211>  95
<212>  PRT
<213>  Homo sapiens

<400>  56

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro
                85                  90                  95

<210>  57
<211>  17
<212>  PRT
<213>  Homo sapiens

<400>  57

Glu Met Gly Gly Ile Thr Gln Thr Pro Tyr Lys Val Ser Ile Ser Gly
1               5                   10                  15

Thr

<210>  58
<211>  4
<212>  PRT
<213>  Homo sapiens

<400>  58

Tyr Gly Met His
1


<210>  59
<211>  5
<212>  PRT
<213>  Homo sapiens

<400>  59

Asp Ser Val Lys Gly
1               5


<210>  60
<211>  16
<212>  PRT
<213>  Homo sapiens


<220>
<221>  MISC_FEATURE
<222>  (4)..(4)
<223>  where X is any amino acid

<220>
<221>  MISC_FEATURE
<222>  (6)..(9)
<223>  where X is any amino acid

<220>
<221>  MISC_FEATURE
<222>  (11)..(11)
<223>  where X is any amino acid

<400>  60

Ile Trp Tyr Xaa Gly Xaa Xaa Xaa Xaa Tyr Xaa Asp Ser Val Lys Gly
1               5                   10                  15


<210>  61
<211>  9
<212>  PRT
<213>  Homo sapiens

<220>
<221>  MISC_FEATURE
<222>  (1)..(2)
<223>  where X is any amino acid

<220>
<221>  MISC_FEATURE
<222>  (5)..(6)
<223>  where X is any amino acid

<220>
<221>  MISC_FEATURE
<222>  (9)..(9)
<223>  where X is any amino acid

<400>  61

Xaa Xaa Gly Tyr Xaa Xaa Phe Asp Xaa
1               5


<210>  62
<211>  9
<212>  PRT
<213>  Homo sapiens

<400>  62

Gly Thr Gly Tyr Asn Trp Phe Asp Pro
1               5


<210>  63
<211>  9
<212>  PRT
<213>  Homo sapiens

<400>  63

Gln Met Gly Tyr Trp His Phe Asp Leu
1               5


<210>  64
<211>  5
<212>  PRT
<213>  Homo sapiens

<400>  64

Val Thr Val Ser Ser
1               5


<210>  65

<211>  8
<212>  PRT
<213>  Homo sapiens

<400>  65

Gly Thr Leu Val Thr Val Ser Ser
1               5


<210>  66
<211>  11
<212>  PRT
<213>  Homo sapiens

<400>  66

Trp Gly Arg Gly Thr Leu Val Thr Val Ser Ser
1               5                   10


<210>  67
<211>  412
<212>  DNA
<213>  Homo sapiens

<400>  67
tgattcatgg agaaatagag agactgagtg tgagtgaaca tgagtgagaa aaactggatt      60

tgtgtggcat tttctgataa cggtgtcctt ctgtttgcag gtgtccagtg tcaggtgcag     120

ctggtggagt ctgggggagg cgtggtccag cctgggaggt ccctgagact ctcctgtgca     180

gcgtctggat tcaccttcag tagctatggc atgcactggg tccgccaggc tccaggcaag     240

gggctggagt gggtggcagt tatatggtat gatggaagta ataaatacta tgcagactcc     300

gtgaagggcc gattcaccat ctccagagac aattccaaga acacgctgta tctgcaaatg     360

aacagcctga gagccgagga cacggctgtg tattactgtg cgagagacac ag             412


<210>  68
<211>  50
<212>  PRT
<213>  Homo sapiens

<400>  68

Val Gln Cys Gln Val Gln Leu Val Glu Ser Gly Gly Gly Val Val Gln
1               5                   10                  15


Pro Gly Arg Ser Leu Arg Leu Ser Cys Ala Ala Ser Gly Phe Thr Phe
            20                  25                  30

Ser Ser Tyr Gly Met His Trp Val Arg Gln Ala Pro Gly Lys Gly Leu
        35                  40                  45

Glu Trp
    50

<210>  69
<211>  17
<212>  PRT
<213>  Homo sapiens

<400>  69

Ala Ile Trp Tyr Asn Gly Arg Lys Gln Asp Tyr Ala Asp Ser Val Lys
1               5                   10                  15

Gly

<210>  70
<211>  17
<212>  PRT
<213>  Homo sapiens

<400>  70

Ile Ile Trp Tyr Asp Gly Ser Lys Lys Asn Tyr Ala Asp Ser Val Lys
1               5                   10                  15

Gly

<210>  71
<211>  17
<212>  PRT
<213>  Homo sapiens

<400>  71

Val Ile Trp Tyr Asp Gly Ser Lys Lys Tyr Tyr Val Asp Ser Val Lys
1               5                   10                  15

Gly

<210>  72
<211>  17

<212>  PRT
<213>  Homo sapiens

<400>  72

Val Ile Trp Tyr Asp Gly Ser Asn Lys Tyr Tyr Ala Asp Ser Val Lys
1               5                   10                  15

Gly

<210>  73
<211>  13
<212>  PRT
<213>  Homo sapiens

<400>  73

Trp Val Arg Gln Ala Pro Gly Lys Gly Leu Glu Trp Val
1               5                   10

<210>  74
<211>  31
<212>  PRT
<213>  Homo sapiens

<400>  74

Arg Phe Thr Ile Ser Arg Asp Asn Ser Lys Asn Thr Leu Tyr Leu Gln
1               5                   10                  15

Met Asn Ser Leu Arg Ala Glu Asp Thr Ala Val Tyr Tyr Cys Ala
            20                  25                  30

## Description

### FIELD OF THE INVENTION

This invention relates to formulation and dosing of anti-CD3 antibodies as well as to methods for use thereof.

### BACKGROUND OF THE INVENTION

Antibodies to the CD3 epsilon signaling molecule of the T-cell receptor complex have proven to be useful as immunosuppressants and in the treatment of autoimmune disorders. Thus, improved methods of preparing anti-CD3 antibodies, methods of purifying anti-CD3 antibodies and pharmaceutical formulations containing anti-CD3 antibodies would be useful.

### SUMMARY OF THE INVENTION

The present invention provides formulation and dosing for monoclonal antibodies specifically directed against CD3. The invention also provides methods of manufacturing anti-CD3 monoclonal antibodies and methods of purifying anti-CD3 antibodies.

The pharmaceutical formulations of an anti-CD3 antibody described herein include a pH buffering agent effective in the range of 3.0 to 6.2; a salt; a surfactant; and pharmaceutically effective quantity of an anti-CD3 antibody.

The salt is, for example, sodium chloride; the surfactant is, *e.g.,* an ionic, anionic or zwitterionic surfactant For example, the surfactant is an ionic surfactant such as a polysorbate, *e.g.,* polysorbate 80. The pH buffering agent includes, for example, sodium acetate. In some embodiments, the pH buffering agent is selected from a sodium citrate/citric acid, and sodium acetate/acetic acid.

The pH buffering agent is effective in a range of 10 mM to 50 mM. The pH buffering agent used in the formulations described herein provides a pH range between 5.0 and 6.0. For example, the pH buffering agent provides a pH range between 5.2 and 5.8. In some embodiments, the pH buffering agent provides a pH range between 5.4 and 5.6. For example, the pH buffering agent provides a pH of about 5.5.

In the formulations described herein, the salt is present in a range of 100 mM to 140 mM. The surfactant is 0.02% by weight/volume. The pharmaceutically effective quantity of the anti-CD3 antibody is formulated to provide a quantity per dose in the range of 0.05 mg to 10 mg of anti-CD3 antibody. In some embodiments, the pharmaceutically effective quantity of the anti-CD3 antibody is formulated to provide a quantity per dose in the range of 0.1 mg to 5.0 mg of anti-CD3 antibody. For example, the pharmaceutically effective quantity of the anti-CD3 antibody is formulated to provide a quantity per dose in the range of 0.5 mg to 3.0 mg of anti-CD3 antibody.

In the anti-CD3 antibody formulations described herein, the anti-CD3 antibody is, e.g., 28F11, 27H5, 23F10, 15C3, Orthoclone OKT3, human OKT3γ1 (HOKT3γ1) or ChAglyCD3.

Anti-CD3 antibody pharmaceutical formulations provided herein include a pH buffering agent comprising sodium acetate effective in the range of 3.0 to 6.2, sodium chloride, a surfactant comprising a polysorbate, and a pharmaceutically effective quantity of an anti-CD3 antibody. The polysorbate is, for example, polysorbate 80. The pH buffering agent is effective in a range of 10 mM to 50 mM. The pH buffering agent used in the formulations described herein provides a pH range between 5.0 and 6.0. For example, the pH buffering agent provides a pH range between 5.2 and 5.8. In some embodiments, the pH buffering agent provides a pH range between 5.4 and 5.6. For example, the pH buffering agent provides a pH of about 5.5.

In the pharmaceutical formulations described herein, the salt is present in a range of 100 mM to 140 mM. The surfactant is 0.02% by weight/volume. The pharmaceutically effective quantity of the anti-CD3 antibody is formulated to provide a quantity per dose in the range of 0.05 mg to 10 mg of anti-CD3 antibody. In some embodiments, the pharmaceutically effective quantity of the anti-CD3 antibody is formulated to provide a quantity per dose in the range of 0.1 mg to 5.0 mg of anti-CD3 antibody. For example, the pharmaceutically effective quantity of the anti-CD3 antibody is formulated to provide a quantity per dose in the range of 0.5 mg to 3.0 mg of anti-CD3 antibody. The anti-CD3 antibody is, e.g., 28F11, 27H5, 23F10, 15C3, Orthoclone OKT3, human OKT3γl (HOKT3γ1) or ChAglyCD3.

In one embodiment of the formulations of an anti-CD3 antibody provided herein, the pharmaceutical formulation contains an effective quantity per dose of anti-CD3 antibody in the range of 0.5 mg to 3.0 mg, between 1 to 3 mg sodium acetate, between 5 to 9 mg of sodium chloride, and between 0.1 to 0.3 micrograms Polysorbate 80, such that the formulation is adjusted to 1.0 mL with water. For example, the pharmaceutical formulation contains 2.05 mg sodium acetate, 7.31 mg sodium chloride and 0.216 micrograms Polysorbate 80. The pH of the pharmaceutical formulation is, e.g., 5.5.

Also provided herein are methods of treating an autoimmune disease or inflammatory disorder in a subject by administering to a subject in need thereof an effective dose of an anti-CD3 antibody formulated to provide a quantity per dose in the range of 0.05 mg to 10 mg of anti-CD3 antibody per day for a period of five days. For example, the effective dose of an anti-CD3 antibody is formulated to provide a quantity per dose in the range of 0.1 mg to 5.0 mg of anti-CD3 antibody per day for a period of five days. Preferably, the effective dose of an anti-CD3 antibody is formulated to provide a quantity per dose in the range of 0.5 mg to 3.0 mg of anti-CD3 antibody per day for a period of five days. In the methods described herein, the anti-CD3 antibody formulation is administered intravenously. For example, the formulation is administered via continuous intravenous infusion.

Also provided herein are methods of treating or preventing transplant rejection in a subject by administering to the subject, after or concurrent with, transplant, an anti-CD3 antibody at an effective dose and increasing the dose each day thereafter until a 50% or greater TCR-CD3 saturation is achieved, followed by 5 daily doses with the total course of treatment not to exceed eight days. In the methods described herein, the anti-CD3 antibody formulation is administered intravenously. For example, the formulation is administered via continuous intravenous infusion. Preferably, the effective dose of anti-CD3 antibody results in a level of cytokine release that is less than 3 on the WHO toxicity grading scale.

The anti-CD3 antibody formulations provided herein are administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

Exemplary monoclonal antibodies include 28F11, 27H5, 23F10, and 15C3 provided herein, as well as Orthoclone OKT3, human OKT3γl (HOKT3γ1) and ChAglyCD3. Preferably, the monoclonal antibody is an antibody that binds to the same epitope as 28F11, 27H5, 23F10 or 15C3. Also preferably, the antibodies are fully human antibodies ("huCD3 antibodies"). The anti-CD3 antibody has one or more of the following characteristics: the antibody binds to CD3 positive (CD3+) cells but not CD3 negative (CD3-) cells; the anti-CD3 antibody induces antigenic modulation which involves alteration (e.g., decrease) of the cell surface expression level or activity of CD3 or me T cell receptor (TcR); the anti-CD3 antibody inhibits binding of the murine anti-human OKT3 monoclonal antibody to T-lymphocytes; or the anti-CD3 antibody binds an epitope of CD3 that wholly or partially includes the amino acid sequence EMGGITQTPYKVSISGT (SEQ ID NO:57). The anti-CD3 antibody competes with the murine anti-CD3 antibody OKT3 for binding to CD3, and exposure to the anti-CD3 antibody removes or masks CD3 and/or TcR without affecting cell surface expression of CD2, CD4 or CD8.

Inhibiting the binding of the murine anti-human OKT3 monoclonal antibody to a T-lymphocyte is defined as a decrease in the ability of the murine OKT3 antibody to form a complex with CD3 on the cell surface of a T-lymphocyte.

An anti-CD3 antibody contains a heavy chain variable having the amino acid sequence of SEQ ID NOS: 2, 6, 10 or 22 and a light chain variable having the amino acid sequence of SEQ ID NOS: 4, 8, 16-20 or 25-26. Preferably, the three heavy chain CDRs include an amino acid sequence at least 90%, 92%, 95%, 97% 98%, 99% or more identical a sequence selected from the group consisting of GYGMH (SEQ ID NO:27); VIWYDGSKKYYVDSVKG (SEQ ID NO:28); QMGYWHFDL (SEQ ID NO:29); SYGMH (SEQ ID NO:33); IIWYDGSKKNYADSVKG (SEQ ID NO:34); GTGYNWFDP (SEQ ID NO:35); and AIWYNGRKQDYADSVKG (SEQ ID NO:44) and a light chain with three CDR that include an amino acid sequence at least 90%, 92%, 95%, 97% 98%, 99% or more identical to a sequence selected from the group consisting of the amino acid sequence of RASQSVSSYLA (SEQ ID NO:30); DASNRAT (SEQ ID NO:31); QQRSNWPPLT (SEQ ID NO:32); RASQSVSSSYLA (SEQ ID NO:36); GASSRAT (SEQ ID NO:37); QQYGSSPIT (SEQ ID NO:38); RASQGISSALA (SEQ ID NO:39); YASSLQS (SEQ ID NO:40); QQYYSTLT (SEQ ID NO:41); DASSLGS (SEQ ID NO:42); WASQGISSYLA (SEQ ID NO:43); QQRSNWPWT (SEQ ID NO:45); DASSLES (SEQ ID NO:46); and QQFNSYPIT (SEQ ID NO:47). The antibody binds CD3.

An anti-CD3 antibody provided herein exhibits at least two or more *(i.e.,* two or more, three or more, four or more, five or more, six or more, seven or more, eight or more, nine or more, ten or more, eleven or more) of the following characteristics: the antibody contains a variable heavy chain region (V_{H}) encoded by a human DP50 V_{H} germline gene sequence, or a nucleic acid sequence that is homologous to the human DP50 V_{H} germline gene sequence; the antibody contains a variable light chain region (V_{L}) encoded by a human L6 V_{L} germline gene sequence, or a nucleic acid sequence homologous to the human L6 V_{L} germline gene sequence; the antibody contains a V_{L} encoded by a human L4/18a V_{L} germline gene sequence, or a nucleic acid sequence homologous to the human L4/18a V_{L} gennline gene sequence; the antibody includes a V_{H} CDR1 region comprising the amino acid sequence YGMH (SEQ ID NO:58); the antibody includes a V_{H} CDR2 region comprising the amino acid sequence DSVKG (SEQ ID NO:59); the antibody includes a V_{H} CDR2 region comprises the amino acid sequence IWYX₁GX₂X₃X₄X₅Y X₆DSVKG (SEQ ID NO:60); the antibody includes a V_{H} CDR3 region comprising the amino acid sequence X_{A}X_{B}GYX_{C}X_{D}FDX_{E} (SEQ ID NO:61); the antibody includes a V_{H} CDR3 region comprising the amino acid sequence GTGYNWFDP (SEQ ID NO:62) or the amino acid sequence QMGYWHFDL (SEQ ID NO:63); the antibody includes the amino acid sequence VTVSS (SEQ ID NO:64) at a position that is C-terminal to the CDR3 region, wherein the position is in a variable region C-terminal to the CDR3 region; the antibody includes the amino acid sequence GTLVTVSS (SEQ ID NO:65) at a position that is C-terminal to CDR3 region, wherein the position is in a variable region C-terminal to the CDR3 region; the antibody includes the amino acid sequence WGRGTLVTVSS (SEQ ID NO:66) at a position that is C-terminal to CDR3 region, wherein the position is in a variable region C-terminal to the CDR3 region; the antibody binds an epitope that wholly or partially includes the amino acid sequence EMGGITQTPYKVSISGT (SEQ ID NO:57); and the antibody includes a mutation in the heavy chain at an amino acid residue at position 234, 235, 265, or 297 or combinations thereof, and wherein the release of cytokines from a T-cell in the presence of said antibody is reduced as compared to the release of cytokines from a T-cell in the presence of an antibody that does not include a mutation in the heavy chain at position 234, 235, 265 or 297 or combinations thereof. The numbering of the heavy chain residues provided herein is that of the EU index *(see* Kabat et al., "Proteins of Immunological Interest", US Dept. of Health & Human Services (1983)), as shown, e.g., in U.S. Patent Nos. 5,624,821 and 5,648,260, the contents of which are hereby incorporated in its entirety by reference.

The anti-CD3 antibody may contain an amino acid mutation. Typically, the mutation is in the constant region. The mutation results in an antibody that has an altered effector function. An effector function of an antibody is altered by altering, i.e., enhancing or reducing, the affinity of the antibody for an effector molecule such as an Fc receptor or a complement component. For example, the mutation results in an antibody that is capable of reducing cytokine release from a T-cell. For example, the mutation is in the heavy chain at amino acid residue 234, 235, 265, or 297 or combinations thereof. Preferably, the mutation results in an alanine residue at either position 234, 235, 265 or 297, or a glutamate residue at position 235, or a combination thereof. The term "cytokine" refers to all human cytokines known within the art that bind extracellular receptors expressed on the cell surface and thereby modulate cell function, including but not limited to IL-2, IFN-gamma, TNF-a, IL-4, IL-5, IL-6, IL-9, IL-10, and IL-13.

Preferably, the anti-CD3 antibody provided herein contains one or more mutations that prevent heavy chain constant region-mediated release of one or more cytokine(s) *in vivo*.

The fully human CD3 antibodies provided herein include, for example, a L²³⁴ L²³⁵ →A²³⁴ E²³⁵ mutation in the Fc region, such that cytokine release upon exposure to the anti-CD3 antibody is significantly reduced or eliminated. As described below in Example 4, the L²³⁴ L²³⁵ → A²³⁴ E²³⁵ mutation in the Fc region of the anti-CD3 antibodies provided herein reduces or eliminates cytokine release when the anti-CD3 antibodies are exposed to human leukocytes, whereas the mutations described below maintain significant cytokine release capacity. For example, a significant reduction in cytokine release is defined by comparing the release of cytokines upon exposure to the anti-CD3 antibody having a L²³⁴ L²³⁵ → A²³⁴ E²³¹ mutation in the Fc region to level of cytokine release upon exposure to another anti-CD3 antibody having one or more of the mutations described below. Other mutations in the Fc region include, for example, L²³⁴ L²³⁵ → A²³⁴ A²³⁵, L²³⁵ → E²³⁵, N²⁹⁷ → A²⁹⁷, and D ²⁶⁵ → A²⁶⁵.

Alternatively, the anti-CD3 antibody is encoded by a nucleic acid that includes one or more mutations that replace a nucleic acid residue with a germline nucleic acid residue. By "germline nucleic acid residue" is meant the nucleic acid residue that naturally occurs in a germline gene encoding a constant or variable region. Thus, the antibodies provided herein include one or more mutations that replace a nucleic acid with the germline nucleic acid residue. Germline antibody genes include, for example, DP50 (Accession number: IMGT/EMBL/GenBank/ DDBJ:L06618), L6 (Accession number: IMGT/EMBL/GenBank/DDBJ:X01668) and L4/18a (Accession number: EMBL/GenBank/DDBJ: Z00006).

The heavy chain of a huCD3 antibody is derived from a germ line V (variable) gene such as, for example, the DP50 germline gene. The nucleic acid and amino acid sequences for the DP50 germline gene include, for example, the nucleic acid and amino acid sequences shown below:

tgattcatgg agaaatagag agactgagtg tgagtgaaca tgagtgagaa aaactggatt
tgtgtggcat tttctgataa cggtgtcctt ctgtttgcag gtgtccagtg tcaggtgcag
ctggtggagt ctgggggagg cgtggtccag cctgggaggt ccctgagact ctcctgtgca
gcgtctggat tcaccttcag tagctatggc atgcactggg tccgccaggc tccaggcaag
gggctggagt gggtggcagt tatatggtat gatggaagta ataaatacta tgcagactcc
gtgaagggcc gattcaccat ctccagagac aattccaaga acacgctgta tctgcaaatg
aacagcctga gagccgagga cacggctgtg tattactgtg cgagagacac ag (SEQ ID
NO: 67)

VQCQVQLVES GGGVVQPGRS LRLSCAASGF TFSSYGMHWV RQAPGKGLEW VAVIWYDGSN
KYYADSVKGR FTISRDNSKN TLYLQMNSLR AEDTAVYYCA R (SEQ ID NO: 68)

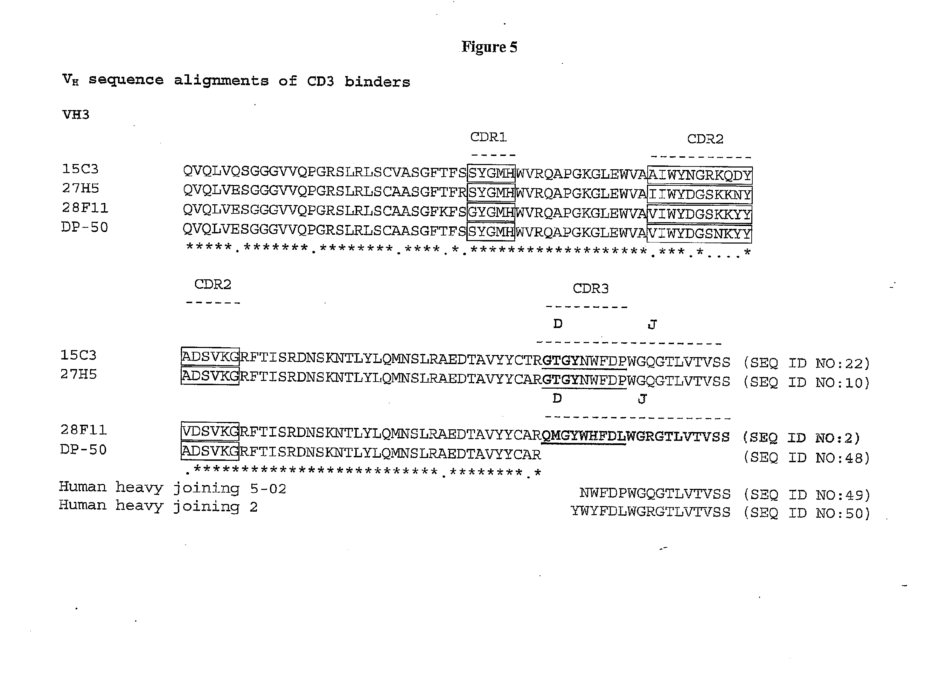

The huCD3 antibodies include a variable heavy chain (V_{H}) region encoded by a human DP50 V_{H} germline gene sequence. A DP50 V_{H} germline gene sequence is shown, e.g., in SEQ ID NO:48 in Figure 5. The huCD3 antibodies provided herein include a V_{H} region that is encoded by a nucleic acid sequence that is at least 80% homologous to the DP50 V_{H} germline gene sequence. Preferably, the nucleic acid sequence is at least 90%, 95%, 96%, 97% homologous to the DP50 V_{H} germline gene sequence, and more preferably, at least 98%, 99% homologous to the DP50 V_{H} germline gene sequence. The V_{H} region of the huCD3 antibody is at least 80% homologous to the amino acid sequence of the V_{H} region encoded by the DP50 V_{H} germline gene sequence. Preferably, the amino acid sequence of V_{H} region of the HuCD3 antibody is at least 90%, 95%, 96%, 97% homologous to the amino acid sequence encoded by the DP50 V_{H} germline gene sequence, and more preferably, at least 98%, 99% homologous to the sequence encoded by the DP50 V_{H} germline gene sequence.

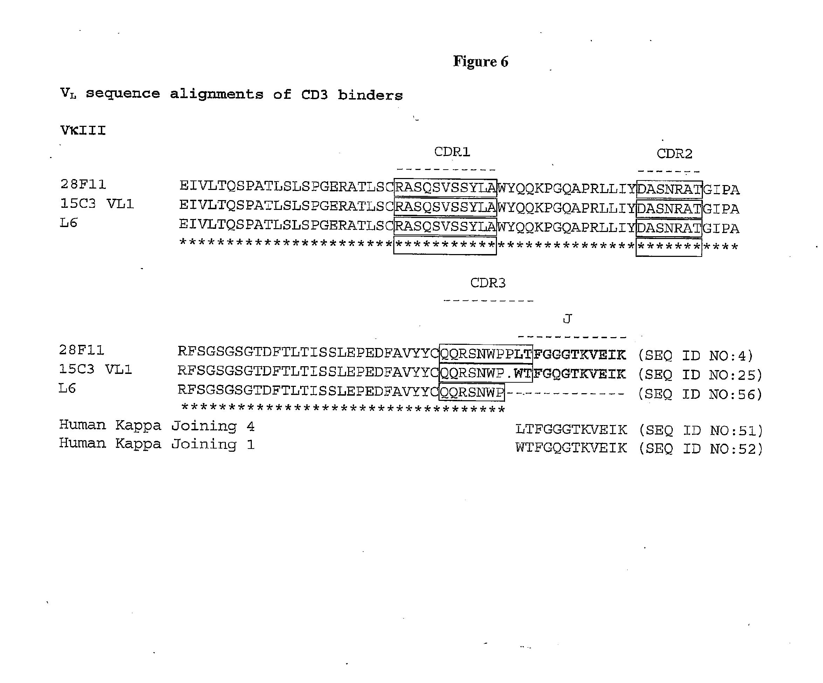

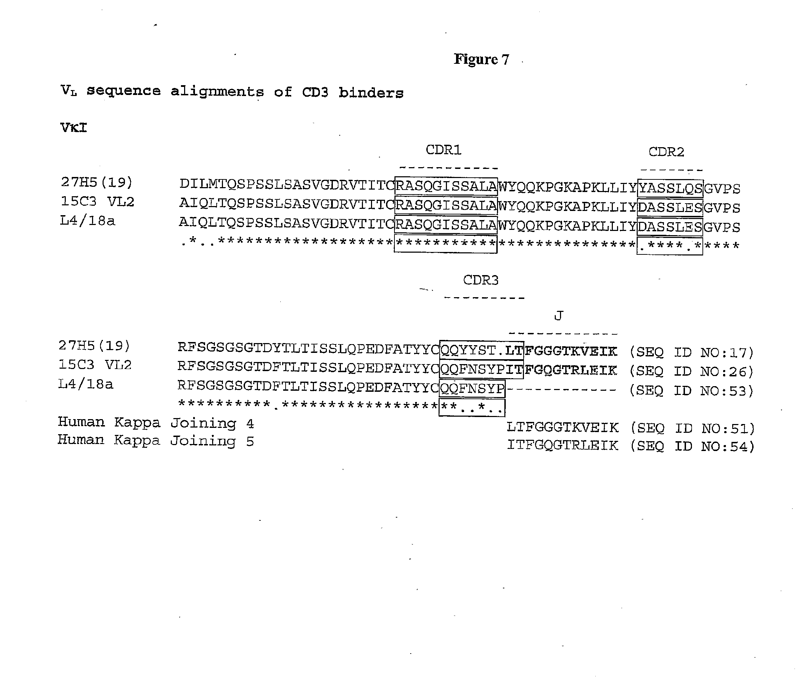

The huCD3 antibodies also include a variable light chain (V_{L}) region encoded by a human L6 or L4/18a V_{L} germline gene sequence. A human L6 V_{L} germline gene sequence is shown, *e.g*., in SEQ ID NO:56 in Figure 6, and a human L4/18a V_{L} germline gene sequence is shown, for example, in SEQ ID NO:53 in Figure 7. Alternatively, the huCD3 antibodies include a V_{L} region that is encoded by a nucleic acid sequence that is at least 80% homologous to either the L6 or L4/18a V_{L} germline gene sequence. Preferably, the nucleic acid sequence is at least 90%, 95%, 96%, 97% homologous to either the L6 or L4/18a V_{L} germline gene sequence, and more preferably, at least 98%, 99% homologous to either the L6 or L4/18a V_{L} germline gene sequence. The V_{L} region of the huCD3 antibody is at least 80% homologous to the amino acid sequence of the V_{L} region encoded by either the L6 or L4/18a V_{L} germline gene sequence. Preferably, the amino acid sequence of V_{L} region of the huCD3 antibody is at least 90%, 95%, 96%, 97% homologous to the amino acid sequence encoded by either the L6 or L4/18a V_{L} germline gene sequence, and more preferably, at least 98%, 99% homologous to the sequence encoded by either the L6 or L4/18a V_{L} germline gene sequence.

The huCD3 antibodies have, for example, partially conserved amino acid sequences that are derived from the DP50 germline. For example, the CDR1 region of huCD3 antibodies used herein have at least the contiguous amino acid sequence YGMH (SEQ ID NO: 58).

The CDR2 of the anti-CD3 antibodies includes, *e.g*., at least the contiguous amino acid sequence DSVKG (SEQ ID NO: 59). For example, the CDR2 region includes the contiguous amino acid sequence FVYX₁GX₂X₃X₄X₅YX₆DSVKG (SEQ ID NO:60), where X₁, X₂, X₃, X₄, X₅ and X₆ represent any amino acid. For example, X₁, X₂, X₃ and X₄ are hydrophilic amino acids. In some anti-CD3 antibodies used herein, X₁ is asparagine or aspartate, X₂ is arginine or serine, X₃ is lysine or asparagine, X₄ is lysine or glutamine, X₅ is aspartate, asparagine or tyrosine, and/or X₆ is valine or alanine. For example, the V_{H} CDR2 region includes an amino acid sequence selected from the group consisting of AIWYNGRKQDYADSVKG (SEQ ID NO:69), IIWYDGSKKNYADSVKG (SEQ ID NO:70), VIWYDGSKKYYVDSVKG (SEQ ID NO:71) and VIWYDGSNKYYADSVKG (SEQ ID NO:72).

The CDR3 region of anti-CD3 antibodies contain, for example, at least the contiguous amino acid sequence X_{A}X_{B}GYX_{C}X_{D}FDX_{E}. (SEQ ID NO:61), where X_{A}, X_{B}, X_{C}, X_{D}, and X_{E} represent any amino acid. In some anti-CD3 antibodies used herein, X_{A} and X_{B} are neutral amino acids, X_{D} is an aromatic amino acid, and/or wherein X_{E} is a hydrophobic amino acid. For example, X_{A} is glycine or glutamine, X_{B} is threonine or methionine, X_{C} is asparagine or tryptophan, X_{D} is tryptophan or histidine, and/or X_{E} is proline or leucine. For example, the CDR3 region includes either the contiguous amino acid sequence GTGYNWFDP (SEQ ID NO:62) or the contiguous amino acid sequence QMGYWHFDL (SEQ ID NO: 63).

The anti-CD3 antibodies include a framework 2 region (FRW2) that contains the amino acid sequence WVRQAPGKGLEWV (SEQ ID NO:73). Anti-CD3 antibodies used herein include a framework 3 region (FRW3) that contains the amino acid sequence FTISRDNSKNTLYLQMNSLRAEDTAVYYCA (SEQ ID NO:74).

Some anti-CD3 antibodies include the contiguous amino acid sequence VTVSS (SEQ ID NO:64) at a position that is C-terminal to CDR3 region. For example, the antibody contains the contiguous amino acid sequence GTLVTVSS (SEQ ID NO:65) at a position that is C-terminal to the CDR3 region. Other anti-CD3 antibodies include the contiguous amino acid sequence WGRGTLVTVSS (SEQ ID NO: 66) at a position that is C-terminal to the CDR3 region. The arginine residue in SEQ ID NO:66 is shown, for example, in the V_{H} sequences for the 28F11 huCD3 antibody (SEQ ID NO:2) and the 23F10 huCD3 antibody (SEQ ID NO:6).

In another aspect, the invention provides methods of treating, preventing or alleviating a symptom of an immune-related disorder by administering an anti-CD3 antibody formulation to a subject. The anti-CD3 antibody formulations provided herein are administered in a dosage in the range between 0.05 mg/day and 10 ing/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day For example, the anti-CD3 antibody formulation is administered to a patient suffering from or predisposed to inflammatory bowel disorder, ulcerative colitis, Crohn's disease, multiple sclerosis, rheumatoid arthritis or Type I diabetes. The formulation is administered, e.g., intravenously. Other routes of administration are contemplated. For example, the anti-CD3 antibody formulations are administered subcutaneously, orally, parenterally, nasally, intramuscularly, or any combination of these routes of administration.

In another aspect, the invention provides methods of treating or preventing transplant rejection by administering to a subject an anti-CD3 antibody at a dosage in the range between 0.05 mg/day and 10 mg/day after transplant and increasing the dosage each day thereafter until a level of 50% or greater TCR-CD3 saturation is achieved, followed by 5 daily doses with the total course of treatment not to exceed eight days. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

For example, the anti-CD3 antibody formulation is used to treat or prevent renal rejection after organ or tissue transplantation. The formulation is administered prophylactically (e.g., prior to an acute rejection episode to prevent an episode), and/or the formulation is used as a treatment for an acute rejection episode following transplantation. The formulation is administered, e.g., intravenously. Other routes of administration are contemplated. For example, the anti-CD3 antibody formulations are administered subcutaneously, orally, parenterally, nasally, intramuscularly, or any combination of these routes of administration.

The anti-CD3 antibody formulations provided herein are stored in appropriate containers, such as vials, such that the dosage per container of anti-CD3 antibody formulation is in the range of 1 to 10 mg/container. For example, the dosage per container of anti-CD3 antibody formulation is in the range of 2 to 5 mg/container. Preferably, the dosage per container is in the range of 3.5 to 4.5 mg/container, e.g., the dosage per container is 4 mg/container.

Optionally, the subject is further administered with a second agent such as, but not limited to, anti-inflammatory compounds or immunosuppressive compounds. Suitable compounds include, but are not limited to methotrexate, cyclosporin A (including, for example, cyclosporin microemulsion), tacrolimus, corticosteroids, statins, type I interferons, Remicade (Infliximab), Enbrel (Etanercept) and Humira (Adalimumab). For example, subjects with Type I diabetes or Latent Autoimmune Diabetes in the Adult (LADA), are also administered a second agent, such as, for example, GLP-1 or a beta cell resting compound *(i.e.,* a compound that reduces or otherwise inhibits insulin release, such as potassium channel openers).

In another aspect, the invention provides methods of purifying an anti-CD3 antibody by affinity chromatography, ion-exchange chromatography and hydroxyapatite chromatography. For example, the affinity chromatography is protein A chromatography. The ion exchange chromatography is, e.g., anion exchange chromatography.

### BRIEF DESCRIPTION OF THE DRAWINGS

Figures 1A-1D are a series of representations of the nucleotide sequence and amino acid sequences for the variable light and variable heavy regions of the huCD3 antibody 28F11, wherein the CDRs are highlighted with boxes.
Figures 2A-2D are a series of representations of the nucleotide sequence and amino acid sequences for the variable light and variable heavy regions of the huCD3 antibody 23F10,
Figures 3A-3D are a series of representations of the nucleotide sequence and amino acid sequences for the variable light and variable heavy regions of the huCD3 antibody 27H5. Figure 3E is an alignment of the five light chains from the clone 27H5, where an asterisk (*) represents a conserved amino acid; a colon (:) represents a conservative mutation; and a period (.) represents a semiconservative mutation.
Figures 4A-4D are a series of representations of the nucleotide sequence and amino acid sequences for the variable light and variable heavy regions of the huCD3 antibody 15C3.
Figure 5 is an alignment depicting the variable heavy chain regions of the 15C3, 27H5 and 28F11 huCD3 antibodies as well as the DP-50 germline sequence, the human heavy joining 5-02 sequence, and the human heavy joining 2 sequence. The CDR regions are indicated for each sequence.
Figure 6 is an alignment depicting the VκIII variable regions of the 15C3 (variable light chain 1, *i.e.,* "VL1") and 28F11 huCD3 antibodies, as well as the L6 germline sequence, the human kappa joining 4 sequence and the human kappa joining 1 sequence. The CDR regions are indicated for each sequence.
Figure 7 is an alignment depicting the VκI variable regions of the 15C3 (variable light chain 2, *i.e.,* "VL2") and 27H5 VL2 huCD3 antibodies, as well as the L4/18a germline sequence, the human kappa joining 4 sequence and the human kappa joining 5 sequence. The CDR regions are indicated for each sequence.
Figure 8 is an alignment depicting the VκII variable regions of the 27H5 VL1 huCD3 antibody and DPK22, as well as human Kappa joining 5 sequence. The CDR regions are indicated for each sequence.

### DETAILED DESCRIPTION

The present invention provides formulations and dosing for monoclonal antibody, e.g., fully human monoclonal antibodies, specific against CD3 epsilon chain (CD3ε). The fully human antibodies provided herein are referred to as huCD3 antibodies.

The formulations provided herein have a pH in the range of 3.0 to 7.0. Preferably, the formulation has a pH in the range of 5.0 to 6.0, and more preferably, the formulation has a pH in the range of 5.2 to 5.8, and most preferably, the formulation has a pH in the range of 5.4 to 5.6 For example, in one embodiment, the formulation has a pH of 5.5.

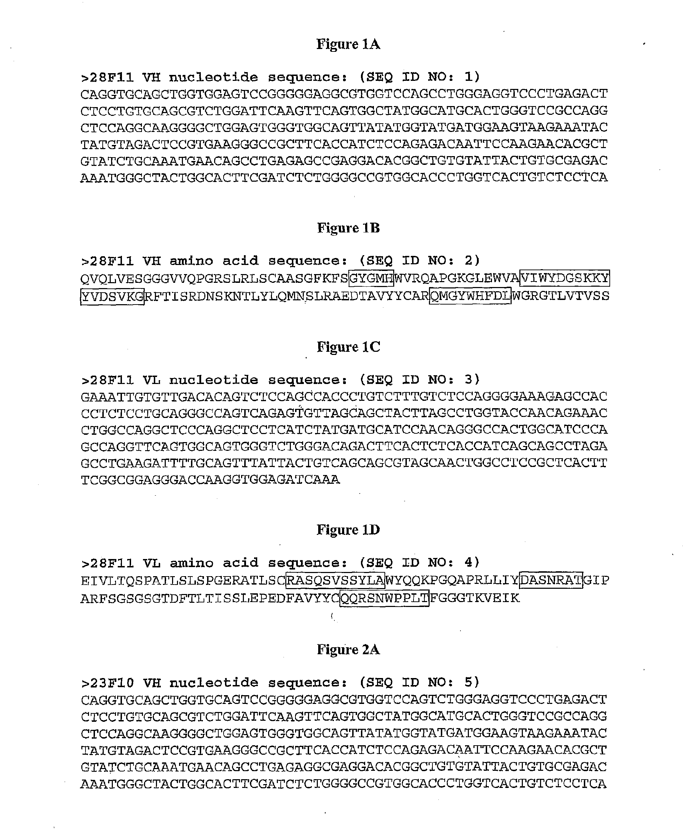

The anti-CD3 antibodies used herein bind to a CD3 that wholly or partially includes the amino acid residues from position 27 to position 43 of the processed human CD3 epsilon subunit *(i.e.,* without the leader sequence). The amino acid sequence of the human CD3 epsilon subunit is shown, for example, in GenBank Accession Nos. NP_000724; AAA52295; P07766; A32069; CAA27516; and AAH49847. For example, the anti-CD3 antibody binds a CD3 epitope that wholly or partially includes the amino acid sequence of EMGGITQTPYKVSISGT (SEQ ID NO: 57). An exemplary huCD3 monoclonal antibody that binds to this epitope is the 28F11 antibody provided herein. The 28F11 antibody includes a heavy chain variable region (SEQ ID NO:2) encoded by the nucleic acid sequence shown below in SEQ ID NO:1, and a light chain variable region (SEQ ID NO:4) encoded by the nucleic acid sequence shown in SEQ ID NO:3 (Figures 1A-1D).

The amino acids encompassing the complementarity determining regions (CDR) as defined by Chothia et al. 1989, E.A. Kabat et al., 1991 are highlighted with boxes in Figures 1B and 1D and Figures 5 and 6. *(See* Chothia, C, et al., Nature 342:877-883 (1989); Kabat, EA, et al., Sequences of Protein of immunological interest, Fifth Edition, US Department of Health and Human Services, US Government Printing Office (1991)). The heavy chain CDRs of the 28F11 antibody have the following sequences: GYGMH (SEQ ID NO:27) VIWYDGSKKYYVDSVKG (SEQ ID NO:28) and QMGYWHFDL (SEQ ID NO:29). The light chain CDRs of the 28F11 antibody have the following sequences: RASQSVSSYLA (SEQ ID NO:30) DASNRAT (SEQ ID NO:31) and QQRSNWPPLT (SEQ ID NO:32).

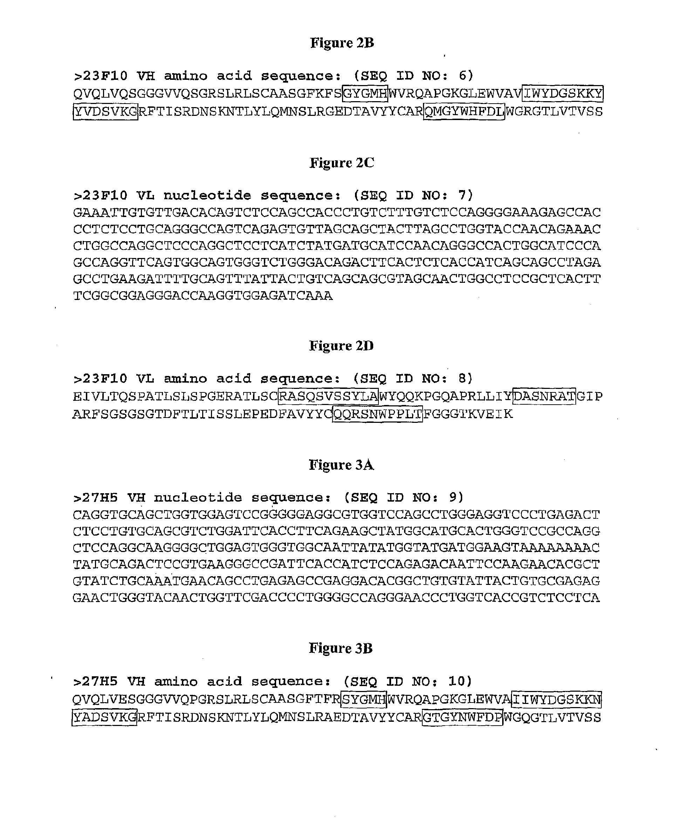

As shown in Figures 2A-2D, the 23F10 antibody includes a heavy chain variable region (SEQ ID NO:6) encoded by the nucleic acid sequence of SEQ ID NO:5, and a light chain variable region (SEQ ID NO:8) encoded by the nucleic acid sequence of SEQ ID NO:7. The amino acids encompassing the CDR as defined by Chothia et al. 1989, E.A. Kabat et al., 1991 are highlighted in Figures 2B, 2D. The heavy chain CDRs of the 23F10 antibody have the following sequences: GYGMH (SEQ ID NO:27) VIWYDGSKKYYVDSVKG (SEQ ID NO:28) and QMGYWHFDL (SEQ ID NO:29). The light chain CDRs of the 23F10 antibody have the following sequences: RASQSVSSYLA (SEQ ID NO:30) DASNRAT (SEQ ID NO:31) and QQRSNWPPLT (SEQ ID NO:32).

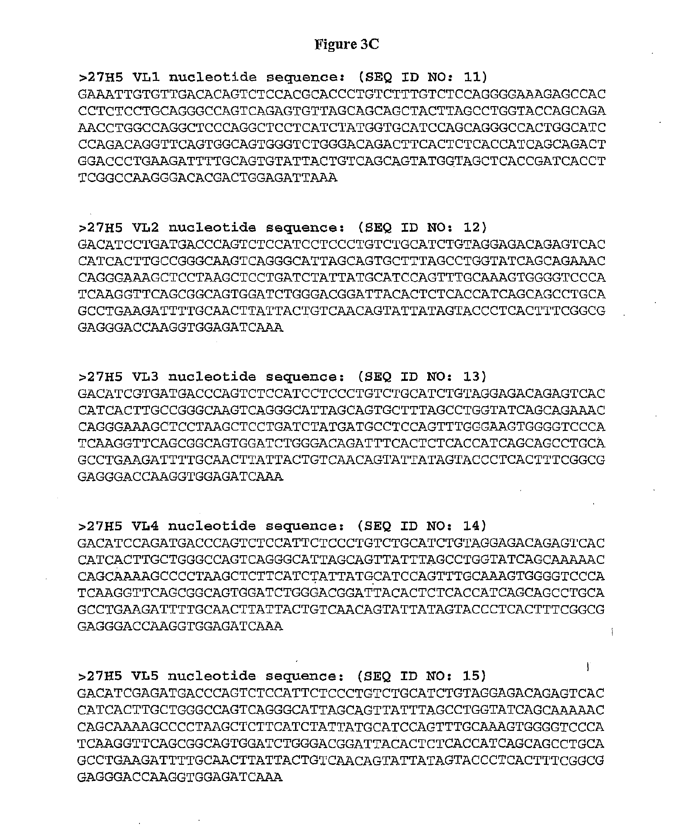

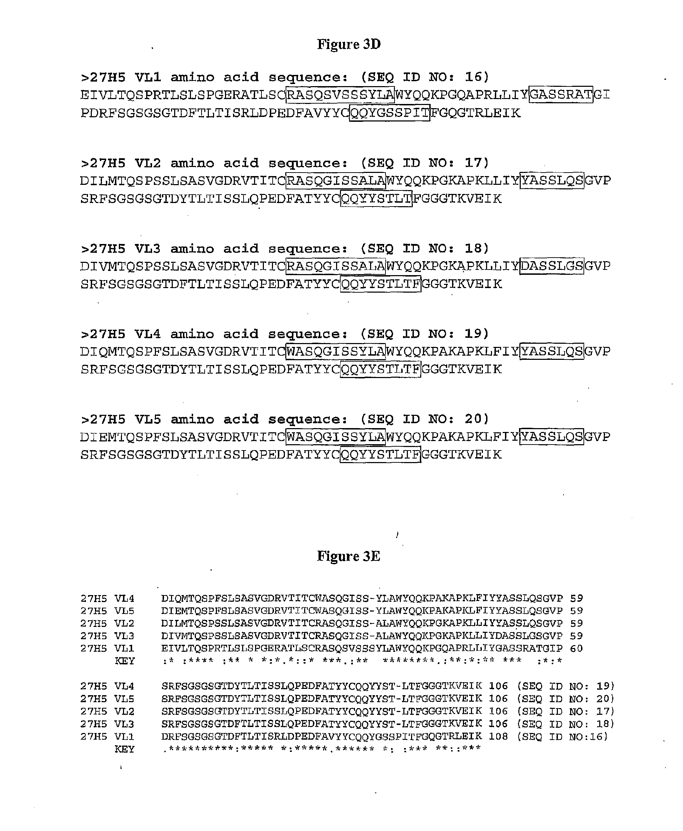

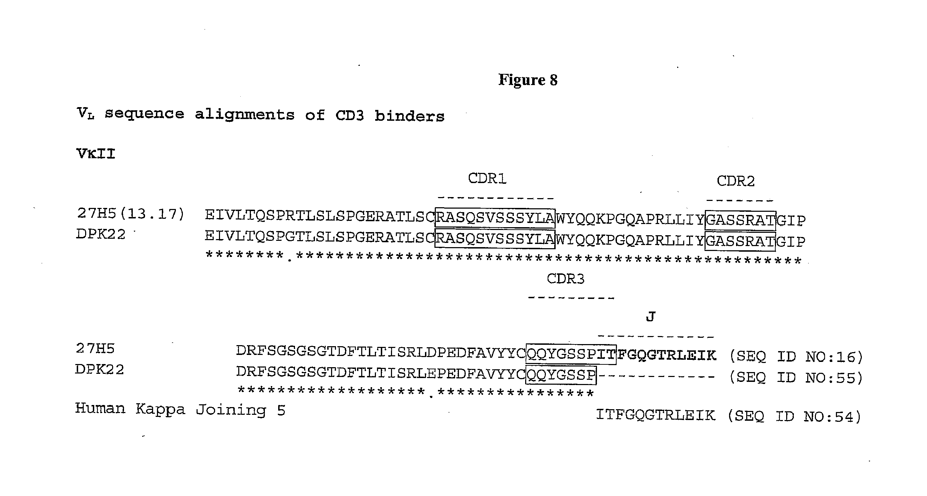

As shown in Figures 3A-3D, the 27H5 antibody includes a heavy chain variable region (SEQ ID NO:10) encoded by the nucleic acid sequence of SEQ ID NO:9, and a light chain variable region selected from the amino acid sequences of SEQ ID NOS: 16-20 and encoded by the nucleic acid sequences of SEQ ID NO:11-15. The amino acids encompassing the CDR as defined by Chothia et al. 1989, E.A. Kabat et al., 1991 are highlighted with boxes in Figures 3B, 3D, 5, and 7-8. The heavy chain CDRs of the 27H5 antibody have the following sequences: SYGMH (SEQ ID NO:33) IIWYDGSKKNYADSVKG (SEQ ID NO:34) and GTGYNWFDP (SEQ ID NO:35). The light chain CDRs of the 27H5 antibody have the following sequences: RASQSVSSSYLA (SEQ ID NO:36); GASSRAT (SEQ ID NO:37); QQYGSSPIT (SEQ ID NO:38); RASQGISSALA (SEQ ID NO:39); YASSLQS (SEQ ID NO:40); QQYYSTLT (SEQ ID NO:41); DASSLGS (SEQ ID NO:42); and WASQGISSYLA (SEQ ID NO:43).

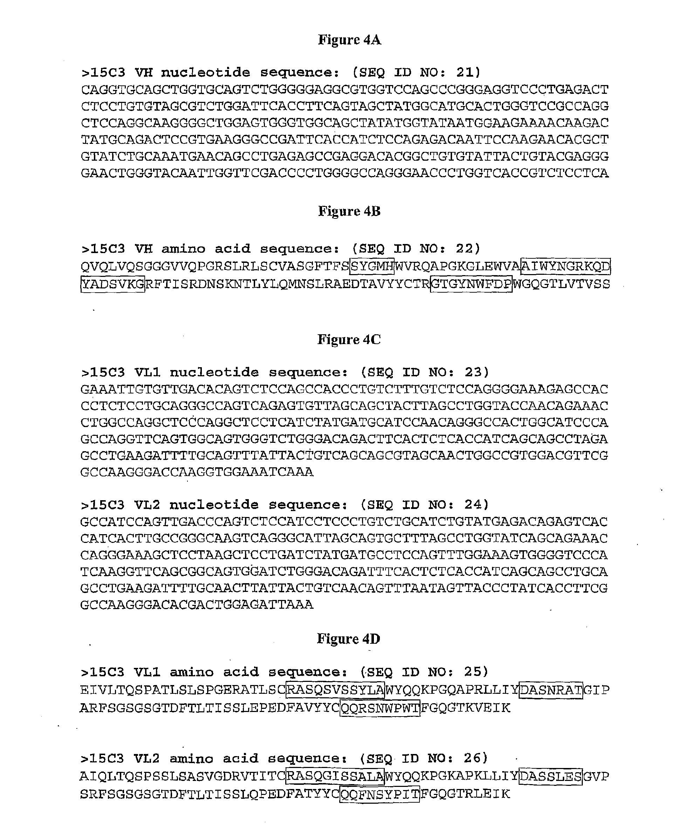

As shown in Figures 4A-4D, the 15C3 antibody includes a heavy chain variable region (SEQ ID NO:22) encoded by the nucleic acid sequence of SEQ ID NO:21, and a light chain variable region selected from the amino acid sequences shown in SEQ ID NOS: 25-26 and encoded by the nucleic acid sequences shown in SEQ ID NO:23-24. The amino acids encompassing the CDR as defined by Chothia et al..1989, E.A. Kabat et al., 1991 are highlighted with boxes in Figures 4B, 4D, and 5-7. The heavy chain CDRs of the 15C3 antibody have the following sequences: SYGMH (SEQ ID NO:33) AIWYNGRKQDYADSVKG (SEQ ID NO:44) and GTGYNWFDP (SEQ ID NO:35). The light chain CDRs of the 15C3 antibody have the following sequences: RASQSVSSYLA (SEQ ID NO:30); DASNRAT (SEQ ID NO:31); QQRSNWPWT (SEQ ID NO:45); RASQGISSALA (SEQ ID NO:39); DASSLES (SEQ ID NO:46); QQFNSYPIT (SEQ ID NO:47).
anti-CD3 antibodies used herein also include antibodies that include a heavy chain variable amino acid sequence that is at least 90%, 92%, 95%, 97% 98%, 99% or more identical the amino acid sequence of SEQ ID NO:2, 6, 10 or 22 and/or a light chain variable amino acid that is at least 90%, 92%, 95%, 97% 98%, 99% or more identical the amino acid sequence of SEQ ID NO:4, 8, 16-20 or 25-26.

Alternatively, the monoclonal antibody is an antibody that binds to the same epitope as 28F11, 27H5, 23F10 or 15C3.

Unless otherwise defined, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (*e.g*., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al. Molecular Cloning: A Laboratory Manual (2d ed., Cold Spring Harbor Laboratory Press, Cold Spring Harbor, N.Y. (1989)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

As used herein, the term "antibody" refers to immunoglobulin molecules and immunologically active portions of immunoglobulin (Ig) molecules, *i.e.,* molecules that contain an antigen binding site that specifically binds (immunoreacts with) an antigen. Such antibodies include, but are not limited to, polyclonal, monoclonal, chimeric, single chain, F_{ab}, F_{ab}, and F_{(ab')2} fragments, and an F_{ab} expression library. By "specifically bind" or "immunoreacts with" is meant that the antibody reacts with one or more antigenic determinants of the desired antigen and does not react (i.e., bind) with other polypeptides or binds at much lower affinity (K_{d} > 10⁻⁶) with other polypeptides.

The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgA, and IgE, respectively. Within light and heavy chains, the variable and constant regions are joined by a "J" region of about 12 or more amino acids, with the heavy chain also including a "D" region of about 10 more amino acids. *See* generally, Fundamental Immunology Ch. 7 (Paul, W., ea., 2nd ed. Raven Press, N.Y. (1989)). The variable regions of each light/heavy chain pair form the antibody binding site.

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs contain an antigen binding site capable of immunoreacting with a particular epitope of the antigen characterized by a unique binding affinity for it.

In general, antibody molecules obtained from humans relate to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain.

As used herein, the term "epitope" includes any protein determinant capable of specific binding to an immunoglobulin, a scFv, or a T-cell receptor. The term "epitope" includes any protein determinant capable of specific binding to an immunoglobulin or T-cell receptor. Epitopic determinants usually consist of chemically active surface groupings of molecules such as amino acids or sugar side chains and usually have specific three dimensional structural characteristics, as well as specific charge characteristics. An antibody is said to specifically bind an antigen when the dissociation constant is ≤1 µM; preferably ≤ 100 nM and most preferably ≤ 10 nM.

As used herein, the terms "immunological binding," and "immunological binding properties" refer to the non-covalent interactions of the type which occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides are quantified using methods well known in the art. One such method entails measuring the rates of antigen-binding site/antigen complex formation and dissociation, wherein those rates depend on the concentrations of the complex partners, the affinity of the interaction, and geometric parameters that equally influence the rate in both directions. Thus, both the "on rate constant" (Kₒₙ) and the "off rate constant" (K_{off}) can be determined by calculation of the concentrations and the actual rates of association and dissociation. *(See* Nature 361:186-87 (1993)). The ratio of K_{off} /Kₒₙ enables the cancellation of all parameters not related to affinity, and is equal to the dissociation constant K_{d}. *(See, generally,* Davies et al. (1990) Annual Rev Biochem 59:439-473). An antibody of the present invention is said to specifically bind to a CD3 epitope when the equilibrium binding constant (K_{d}) is ≤1 µM, preferably ≤ 100 nM, more preferably ≤ 10 nM, and most preferably ≤ 100 pM to about 1 pM, as measured by assays such as radioligand binding assays or similar assays known to those skilled in the art.

Those skilled in the art will recognize that it is possible to determine, without undue experimentation, if a human monoclonal antibody has the same specificity as a human monoclonal antibody used herein (e.g., monoclonal antibody 28F11, 27H5, 23F10 or 15C3) by ascertaining whether the former prevents the latter from binding to a CD3 antigen polypeptide. If the human monoclonal antibody being tested competes with a human monoclonal antibody used herein, as shown by a decrease in binding by the human monoclonal antibody used herein, then the two monoclonal antibodies bind to the same, or a closely related, epitope. Another way to determine whether a human monoclonal antibody has the specificity of a human monoclonal antibody used herein is to pre-incubate the human monoclonal antibody used herein with the CD3 antigen polypeptide with which it is normally reactive, and then add the human monoclonal antibody being tested to determine if the human monoclonal antibody being tested is inhibited in its ability to bind the CD3 antigen polypeptide. If the human monoclonal antibody being tested is inhibited then, in all likelihood, it has the same, or functionally equivalent, epitopic specificity as the monoclonal antibody used herein.

The term "sequence identity" means that two polynucleotide or amino acid sequences are identical *(i.e.,* on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base *(e.g.,* A, T, C, G, U or I) or residue occurs in both sequences to yield the number of matched positions, dividing the number of matched positions by the total number of positions in the comparison window *(i.e.,* the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

As used herein, the twenty conventional amino acids and their abbreviations follow conventional usage. *See* Immunology - A Synthesis (2nd Edition, E.S. Golub and D.R. Gren, Eds., Sinauer Associates, Sunderland7 Mass. (1991)). Stereoisomers (e.g., D-amino acids) of the twenty conventional amino acids, unnatural amino acids such as α-, α-disubstituted amino acids, N-alkyl amino acids, lactic acid, and other unconventional amino acids may also be suitable components for, polypeptides of the present invention. Examples of unconventional amino acids include: 4 hydroxyproline, γ-carboxyglutamate, ε-N,N,N-trimethyllysine, ε-N-acetyllysine, O-phosphoserine, N- acetylserine, N-formylmethionine, 3-methylhistidine, 5-hydroxylysine, σ-N-methylarginine, and other similar amino acids and imino acids *(e.g.,* 4- hydroxyproline). In the polypeptide notation used herein, the lefthand direction is the amino terminal direction and the righthand direction is the carboxy-terminal direction, in accordance with standard usage and convention.

Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide- containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur- containing side chains is cysteine and methionine. Preferred conservative amino acids substitution groups are: valine-leucine-isoleucine, phenylalanine-tyrosine, lysine-arginine, alanine valine, glutamic- aspartic, and asparagine-glutamine.

As discussed herein, minor variations in the amino acid sequences of antibodies or immunoglobulin molecules are contemplated as being encompassed by the present invention, providing that the variations in the amino acid sequence maintain at least 75%, more preferably at least 80%, 90%, 95%, and most preferably 99%. In particular, conservative amino acid replacements are contemplated. Conservative replacements are those that take place within a family of amino acids that are related in their side chains. Genetically encoded amino acids are generally divided into families: (1) acidic amino acids are aspartate, glutamate; (2) basic amino acids are lysine, arginine, histidine; (3) non-polar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. The hydrophilic amino acids include arginine, asparagine, aspartate, glutamine, glutamate, histidine, lysine, serine, and threonine. The hydrophobic amino acids include alanine, cysteine, isoleucine, leucine, methionine, phenylalanine, proline, tryptophan, tyrosine and valine. Other families of amino acids include (i) serine and threonine, which are the aliphatic-hydroxy family; (ii) asparagine and glutamine, which are the amide containing family; (iii) alanine, valine, leucine and isoleucine, which are the aliphatic family; and (iv) phenylalanine, tryptophan, and tyrosine, which are the aromatic family.

The term "agent" is used herein to denote a chemical compound, a mixture of chemical compounds, a biological macromolecule, or an extract made from biological materials.

The term patient includes human and veterinary subjects.

The invention also includes Fᵥ, F_{ab}, F_{ab'} and F_{(ab')2} anti-CD3 fragments, single chain anti-CD3 antibodies, bispecific anti-CD3 antibodies, heteroconjugate anti-CD3 antibodies, trispecific antibodies, immunoconjugates and fragments thereof.

Bispecific antibodies are antibodies that have binding specificities for at least two different antigens. In the present case, one of the binding specificities is for CD3. The second binding target is any other antigen, and advantageously is a cell-surface protein or receptor or receptor subunit.

### Therapeutic Administration and Formulations

It will be appreciated that administration of therapeutic entities in accordance with the invention will be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences (15th ed, Mack Publishing Company, Easton, PA (1975)), particularly Chapter 87 by Blaug, Seymour, therein. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies in accordance with the present invention, provided that the active ingredient in the formulation is not inactivated by the formulation and the formulation is physiologically compatible and tolerable with the route of administration. See also Baldrick P. "Pharmaceutical excipient development: the need for preclinical guidance." Regul. Toxicol Pharmacol. 32(2):210-8 (2000), Wang W. "Lyophilization and development of solid protein pharmaceuticals." Int. J. Phann. 203(1-2):1-60 (2000), Charman WN "Lipids, lipophilic drugs, and oral drug delivery-some emerging concepts." J Pharm Sci.89(8):967-78 (2000), Powell et al. "Compendium of excipients for parenteral formulations" PDA J Pharm Sci Technol. 52:238-311 (1998) and the citations therein for additional information related to formulations, excipients and carriers well known to pharmaceutical chemists.

The formulations are, preferably, substantially pure. As used herein, "substantially pure" means an object species is the predominant species present (*i.e*., on a molar basis it is more abundant than any other individual species in the composition), and preferably a substantially purified fraction is a composition wherein the object species comprises at least about 50 percent (on a molar basis) of all macromolecular species present.

Generally, a substantially pure composition will comprise more than about 80 percent of all macromolecular species present in the composition, more preferably more than about 85%, 90%, 95%, and 99%. Most preferably, the object species is purified to essential homogeneity (contaminant species cannot be detected in the composition by conventional detection methods) wherein the composition consists essentially of a single macromolecular species.

Therapeutic formulations provided herein, which include an anti-CD3 antibody used herein, are used to treat or alleviate a symptom associated with an immune-related disorder, such as, for example, an autoimmune disease or an inflammatory disorder.

Autoimmune diseases include, for example, Acquired Immunodeficiency Syndrome (AIDS, which is a viral disease with an autoimmune component), alopecia areata, ankylosing spondylitis, antiphospholipid syndrome, autoimmune Addison's disease, autoimmune hemolytic anemia, autoimmune hepatitis, autoimmune inner ear disease (AIED), autoimmune lymphoproliferative syndrome (ALPS), autoimmune thrombocytopenic purpura (ATP), Behcet's disease, cardiomyopathy, celiac sprue-dermatitis hepetiformis; chronic fatigue immune dysfunction syndrome (CFIDS), chronic inflammatory demyelinating polyneuropathy (CIPD), cicatricial pemphigold, cold agglutinin disease, crest syndrome, Crohn's disease, Degos' disease, dermatomyositis-juvenile, discoid lupus, essential mixed cryoglobulinemia, fibromyalgia-fibromyositis, Graves' disease, Guillain-Barré syndrome, Hashimoto's thyroiditis, idiopathic pulmonary fibrosis, idiopathic thrombocytopenia purpura (ITP), IgA nephropathy, insulin-dependent diabetes mellitus (Type I diabetes), juvenile chronic arthritis (Still's disease), juvenile rheumatoid arthritis, Ménière's disease, mixed connective tissue disease, multiple sclerosis, myasthenia gravis, pernacious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomena, Reiter's syndrome, rheumatic fever, rheumatoid arthritis, sarcoidosis, scleroderma (progressive systemic sclerosis (PSS), also known as systemic sclerosis (SS)), Sjögren's syndrome, stiff-man syndrome, systemic lupus erythematosus, Takayasu arteritis, temporal arteritis/giant cell arteritis, ulcerative colitis, uveitis, vitiligo and Wegener's granulomatosis.

Inflammatory disorders, include, for example, chronic and acute inflammatory disorders. Examples of inflammatory disorders include Alzheimer's disease, asthma, atopic allergy, allergy, atherosclerosis, bronchial asthma, eczema, glomerulonephritis, graft vs. host disease, hemolytic anemias, osteoarthritis, sepsis, stroke, transplantation of tissue and organs, vasculitis, diabetic retinopathy and ventilator induced lung injury.

The formulations of anti-CD3 antibody are administered to a subject suffering from an immune-related disorder, such as an autoimmune disease or an inflammatory disorder. A subject suffering from an autoimmune disease or an inflammatory disorder is identified by methods known in the art. For example, subjects suffering from an autoimmune disease such as Crohn's disease, ulcerative colitis or inflammatory bowel disease, are identified using any of a variety of clinical and/or laboratory tests such as, physical examination, radiologic examination, and blood, urine and stool analysis to evaluate immune status. For example, patients suffering from multiple sclerosis are identified , e.g., by using magnetic resonance imaging the presence of central nervous system (CNS) lesions that are disseminated in time and space (i.e., occur in different parts of the CNS at least three months apart). Patients suffering from rheumatoid arthritis are identified using, *e.g*., blood tests and/or x-ray or other imaging evaluation. Patients suffering from Type I diabetes are identified, e.g., when any three of these tests is positive, followed by a second positive test on a different day: (1) fasting plasma glucose of greater than or equal to 126 mg/dl with symptoms of diabetes; (2) casual plasma glucose (taken at any time of the day) of greater than or equal to 200 mg/dl with the symptoms of diabetes; or (3) oral glucose tolerance test (OGTT) value of greater than or equal to 200 mg/dl measured at a two-hour interval (the OGTT is given over a three-hour time span).

Administration of an anti-CD3 antibody formulation to a patient suffering from an immune-related disorder such as an autoimmune disease or an inflammatory disorder is considered successful if any of a variety of laboratory or clinical results is achieved. For example, administration of an anti-CD3 antibody formulation to a patient suffering from an immune-related disorder such as an autoimmune disease or an inflammatory disorder is considered successful if one or more of the symptoms associated with the disorder is alleviated, reduced, inhibited or does not progress to a further, *i.e.,* worse, state. Administration of an anti-CD3 antibody formulation to a patient suffering from an immune-related disorder such as an autoimmune disease or an inflammatory disorder is considered successful if the disorder, *e.g*., an autoimmune disorder, enters remission or does not progress to a further, *i.e.*, worse, state.

The anti-CD3 antibody formulations provided herein are used in the treatment, diagnosis and/or prevention of inflammatory bowel disorder (IBD). IBD is the chronic inflammation and irritation of tissue in the gastrointestinal (GI) tract. IBD is associated with symptoms such as abdominal cramping and pain, diarrhea, rectal bleeding, fever and elevated white blood cell count. The anti-CD3 antibody formulations provided herein are administered to a subject that is suffering from, has been diagnosed with, or is predisposed to IBD. The anti-CD3 antibody formulations provided herein are administered at a dosage that is sufficient to alleviate at least one symptom of IBD, to treat IBD, to prevent IBD, and/or to prevent IBD from progressing to a further disease state in a subject. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-GD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

In another embodiment, the anti-CD3 antibody formulations provided herein are used in the treatment, diagnosis and/or prevention of ulcerative colitis. Ulcerative colitis is the chronic inflammation and irritation of the colon. Ulcerative colitis is associated with symptoms such as anemia; fatigue; weight loss; loss of appetite; rectal bleeding; loss of body fluids and nutrients; skin lesions; joint pain; and growth failure (specifically in children). The anti-CD3 antibody formulations provided herein are administered to a subject that is suffering from, has been diagnosed with, or is predisposed to ulcerative colitis. The anti-CD3 antibody formulations provided herein are administered at a dosage that is sufficient to alleviate at least one symptom of ulcerative colitis, to treat ulcerative colitis, to prevent ulcerative colitis, and/or to prevent ulcerative colitis from progressing to a further disease state in a subject. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

In another embodiment, the anti-CD3 antibody formulations provided herein are used in the treatment, diagnosis and/or prevention of Crohn's disease. Crohn's disease is the chronic inflammation and irritation of the intestines. Crohn's disease is associated with symptoms such as abdominal pain, diarrhea, weight loss, poor appetite, fever, night sweats, rectal pain, and rectal bleeding. The anti-CD3 antibody formulation provided herein are administered to a subject that is suffering from, has been diagnosed with, or is predisposed to Crohn's disease. The anti-CD3 antibody formulations provided herein are administered at a dosage that is sufficient to alleviate at least one symptom of Crohn's disease, to treat Crohn's disease, to prevent Crohn's disease, and/or to prevent Crohn's disease from progressing to a further disease state in a subject. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day,1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

In another embodiment, the anti-CD3 antibody formulations provided herein are used in the treatment, diagnosis and/or prevention of multiple sclerosis (MS). MS is a chronic, inflammatory disease that affects the central nervous system (CNS). Symptoms of MS include, for example, changes in sensation, visual problems, muscle weakness, depression, difficulties with coordination and speech, and pain. The anti-CD3 antibody formulations provided herein are administered to a subject that is suffering from, has been diagnosed with, or is predisposed to MS. The anti-CD3 antibody formulations provided herein are administered at a dosage that is sufficient to alleviate at least one symptom of MS, to treat MS, to prevent MS, and/or to prevent MS from progressing to a further disease state in a subject. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

In another embodiment, the anti-CD3 antibody formulations provided herein are used in the treatment, diagnosis and/or prevention of insulin-dependent diabetes mellitus (Type I diabetes). Type I diabetes is a disease characterized by persistent hyperglycemia (high blood sugar levels) resulting from inadequate secretion of the hormone insulin. Type I diabetes is characterized by loss of the insulin-producing beta cells of the islets of Langerhans of the pancreas. Type I diabetes is an autoimmune disorder, in which the body's own immune system attacks the beta cells in the Islets of Langerhans of the pancreas, destroying them or damaging them sufficiently to reduce or eliminate insulin production. Symptoms of Type I diabetes include, for example, increased thirst, increased urination, weight loss despite increased appetite, nausea, vomiting, abdominal pain, and fatigue. The anti-CD3 antibody formulations provided herein are administered to a subject that is suffering from, has been diagnosed with, or is predisposed to Type I diabetes. The anti-CD3 antibody formulations provided herein are administered at a dosage that is sufficient to alleviate at least one symptom of Type I diabetes, to treat Type I diabetes, to prevent Type I diabetes, and/or to prevent Type I diabetes from progressing to a further disease state in a subject. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

In another embodiment, the anti-CD3 antibody formulations provided herein are used in the treatment, diagnosis and/or prevention of rheumatoid arthritis (RA). Rheumatoid arthritis is an autoimmune disease that causes chronic inflammation of the joints. Rheumatoid arthritis can also cause inflammation of the tissue around the joints, as well as other organs in the body. RA is associated with symptoms such as fatigue, lack of appetite, low grade fever, muscle and joint aches, and stiffness. The anti-CD3 antibody formulations provided herein are administered to a subject that is suffering from, has been diagnosed with, or is predisposed to RA. The anti-CD3 antibody formulations provided herein are administered at a dosage that is sufficient to alleviate at least one symptom of RA, to treat RA, to prevent RA, and/or to prevent RA from progressing to a further disease state in a subject. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

The present invention also provides methods of treating or alleviating a symptom associated with an immune-related disorder or a symptom associated with rejection following organ transplantation. For example, the formulations used herein are used to treat or alleviate a symptom of any of the autoimmune diseases and inflammatory disorders provided herein.

The therapeutic formulations used herein are also used as immunosuppression agents in organ or tissue transplantation. As used herein, "immunosuppression agent" refers to an agent whose action on the immune system leads to the immediate or delayed reduction of the activity of at least one pathway involved in an immune response, whether this response is naturally occurring or artificially triggered, whether this response takes place as part of the innate immune system, the adaptive immune system, or both. These immunosuppressive anti-CD3 antibody formulations are administered to a subject prior to, during and/or after organ or tissue transplantation. For example, an anti-CD3 antibody formulation provided herein is used to treat or prevent rejection after organ or tissue transplantation. For example, the anti-CD3 antibody formulation is administered in a dosage in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

Preferably, the therapeutic anti-CD3 antibody formulations provided herein are administered to a subject intravenously or subcutaneously. Other routes of administration are contemplated. For example, the anti-CD3 antibody formulations are administered intravenously, subcutaneously, orally, parenterally, nasally, intramuscularly, or any combination of these routes of administration.

In one embodiment, the anti-CD3 antibody formulations used herein are administered in conjunction with a second agent such as, for example, GLP-1 or a beta cell resting compound (*i.e.,* a compound that reduces or otherwise inhibits insulin release, such as potassium channel openers). Examples of suitable GLP-1 compounds are described in e.g., the published application U.S. 20040037826, and suitable beta cell resting compounds are described in published application U.S. 20030235583, each of which is hereby incorporated by reference in its entirety.

In another embodiment, the anti-CD3 antibody formulations used to treat an immune-related disorder are administered in combination with any of a variety of known anti-inflammatory and/or immunosuppressive compounds. Suitable known compounds include, but are not limited to methotrexate, cyclosporin A (including, for example, cyclosporin microemulsion), tacrolimus, corticosteroids, statins, interferon beta, Remicade (Infliximab), Enbrel (Etanercept) and Humira (Adalimumab).

For example, in the treatment of rheumatoid arthritis, the anti-CD3 antibody formulations used herein can be co-administered with corticosteroids, methotrexate, cyclosporin A, statins, Remicade (Infliximab), Enbrel (Etanercept) and/or Humira (Adalimumab).

In the treatment of uveitis, the anti-CD3 antibody formulations can be administered in conjunction with, *e.g*., corticosteroids, methotrexate, cyclosporin A, cyclophosphamide and/or statins. Likewise, patients afflicted with a disease such as Crohn's Disease or psoriasis can be treated with a combination of an anti-CD3 antibody composition used herein and Remicaid (Infliximab), and/or Humira (Adalimumab).

Patients with multiple sclerosis can receive a combination of an anti-CD3 antibody composition used herein in combination with, *e.g*., glatiramer acetate (Copaxone), interferon beta-1a (Avonex), interferon beta-1a (Rebif), interferon beta-1b (Betaseron or Betaferon), mitoxantrone (Novantrone), dexamethasone (Decadron), methylprednisolone (Depo-Medrol), and/or prednisone (Deltasone) and/or statins.

In one embodiment, the immunosuppressive anti-CD3 antibody formulations used herein are administered in conjunction with a second agent such as, for example, GLP-1 or a beta cell resting compound, as described above.

In another embodiment, these immunosuppressive anti-CD3 antibody formulations are administered in combination with any of a variety of known anti-inflammatory and/or immunosuppressive compounds. Suitable anti-inflammatory and/or immunosuppressive compounds for use with the anti-CD3 antibodies used herein include, but are not limited to, methotrexate, cyclosporin A (including, for example, cyclosporin microemulsion), tacrolimus, corticosteroids and statins.

In yet another embodiment used herein, an anti-CD3 antibody composition is administered to a human individual upon detection of the presence of auto-reactive antibodies within the human individual. Such auto-reactive antibodies are known within the art as antibodies with binding affinity to one or more proteins expressed endogenously within the human individual. In one aspect used herein, the human individual is tested for the presence of auto-reactive antibodies specifically involved in one or more autoimmune diseases as are well known within the art. In one specific embodiment, a human patient is tested for the presence of antibodies against insulin, glutamic acid decarboxylase and/or the IA-2 protein, and subsequently administered with an anti-CD3 antibody upon positive detection of one or more such auto-reactive antibodies.

In another embodiment used herein, an anti-CD3 antibody composition is administered to human subjects to prevent, reduce or decrease the recruitment of immune cells into human tissues. An anti-CD3 antibody used herein is administered to a subject in need thereof to prevent and treat conditions associated with abnormal or deregulated immune cell recruitment into tissue sites of human disease.

In another embodiment used herein, an anti-CD3 antibody composition is administered to human subjects to prevent, reduce or decrease the extravasation and diapedesis of immune cells into human tissues. Thus, the anti-CD3 antibodies used herein are administered to prevent and/or treat conditions associated with abnormal or deregulated immune cell infiltration into tissue sites of human disease.

In another embodiment used herein, an anti-CD3 antibody composition is administered to human subjects to prevent, reduce or decrease the effects mediated by the release of cytokines within the human body. The term "cytokine" refers to all human cytokines known within the art that bind extracellular receptors upon the cell surface and thereby modulate cell function, including but not limited to IL-2, IFN-g, TNF-a, IL-4, IL-5, IL-6, IL-9, IL-10, and IL-13.

In another embodiment used herein, an anti-CD3 antibody composition is administered to human subjects to prevent, reduce or decrease the effects mediated by the release of cytokine receptors within the human body. The term "cytokine receptor" refers to all human cytokine receptors within the art that bind one or more cytolcine(s), as defined herein, including but not limited to receptors of the aforementioned cytokines. Thus, an anti-CD3 antibody used herein is administered to treat and/or prevent conditions mediated through abnormal activation, binding or ligation of one or more cytokine receptor(s) within the human body. It is further envisioned that administration of the anti-CD3 antibody in vivo will deplete the intracellular signaling mediated by cytokine receptor(s) within such human subject.

In one aspect used herein, an anti-CD3 antibody composition is administered to a human individual upon decrease of pancreatic beta-cell function therein. In one embodiment, the individual is tested for beta-cell function, insulin secretion or c-peptide levels as are known within the art. Subsequently, upon notice of sufficient decrease of either the indicator, the human individual is administered with a sufficient dosage regimen of an anti-CD3 antibody to prevent further progression of autoimmune destruction of beta-cell function therein.

### Diagnostic and Prophylactic Formulations

The anti-CD3 antibody formulations (also referred to herein as antibody compositions) provided herein are used in diagnostic and prophylactic formulations. In one embodiment, an anti-CD3 MAb formulation provided herein is administered to patients that are at risk of developing one of the aforementioned autoimmune diseases. A patient's predisposition to one or more of the aforementioned autoimmune diseases can be determined using genotypic, serological or biochemical markers. For example, the presence of particular HLA subtypes and serological autoantibodies (against insulin, GAD65 and IA-2) are indicative of Type I diabetes.

In another embodiment provided herein, an anti-CD3 antibody formulation is administered to human individuals diagnosed with one or more of the aforementioned autoimmune diseases. Upon diagnosis, an anti-CD3 antibody is administered to mitigate or reverse the effects of autoimmunity. In one such example, a human individual diagnosed with Type I diabetes is administered with sufficient dose of an anti-CD3 antibody to restore pancreatic function and minimize damage of autoimmune infiltration into the pancreas. In another embodiment, a human individual diagnosed with rheumatoid arthritis is administered with an anti-CD3 antibody to reduce immune cell infiltration into and destruction of limb joints.

Preferably, the therapeutic, diagnostic and/or prophylactic anti-CD3 antibody formulations provided herein are administered to a subject intravenously or subcutaneously. Other routes of administration are contemplated. For example, the anti-CD3 antibody formulations are administered intravenously, subcutaneously, orally, parenterally, nasally, intramuscularly, or any combination of these routes of administration.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLES

The following examples, including the experiments conducted and results achieved are provided for illustrative purposes only and are not to be construed as limiting upon the present invention.

### EXAMPLE 1: Formulation Development and Analysis

In the formulation study provided herein, the stability of an anti-CD3 antibody in 20 formulations was monitored at defined temperatures over a period of three months. The formulations studied comprised acetate, citrate and phosphate buffering agents covering a pH range of pH 4.0 to 8.0 and included selected excipients Tween 80, mannitol, EDTA, sodium chloride and sucrose. The formulations investigated in the study are shown in the table below:

**Table 1: Formulations investigated**

| **Formulation Code** | **Composition** | **pH** |
|---|---|---|
| F1 | 25mM sodium acetate / 125mM sodium chloride | 4.0 |
| F2 | 25mM sodium acetate / 125mM sodium chloride | 5.5 |
| F3 | 25mM sodium acetate / 125mM sodium chloride / 0.02% Tween 80 | 5.5 |
| F4 | 10mM sodium acetate/ 140mM sodium chloride | 5.5 |
| F5 | 10mM sodium acetate / 100mM sodium chloride / 2% mannitol | 5.5 |
| F6 | 25mM sodium citrate / 125mM sodium chloride | 5.5 |
| F7 | 25mM sodium citrate / 125mM sodium chloride | 6.0 |
| F8 | 25mM sodium citrate / 125mM sodium chloride | 6.5 |
| F9 | 25mM sodium citrate / 125mM sodium chloride / 0.02% Tween 80 | 6.0 |
| F10 | 10mM sodium citrate / 140mM sodium chloride | 6.0 |
| F11 | 10mM sodium citrate/ 100mM sodium chloride/2% mannitol | 6.0 |
| F12 | 25mM sodium citrate / 200mM sucrose | 6.0 |
| F13 | 50mM sodium phosphate /100mM sodium chloride | 6.0 |
| F14 | 50mM sodium phosphate / 100mM sodium chloride | 7.0 |
| F15 | 50mM sodium phosphate / 100mM sodium chloride | 8.0 |
| F16 | 50mM sodium phosphate / 100mM sodium chloride / 0.02% Tween 80 | 7.0 |
| F17 | 50mM sodium phosphate / 100mM sodium m chloride / 0.01% EDTA | 7.0 |
| F18 | 10mM sodium phosphate / 150mM sodium chloride | 7.0 |
| F19 | 10mM sodium phosphate / 100mM sodium chloride / 2% mannitol) | 7.0 |
| F20 | 25mM sodium phosphate / 200mM sucrose | 7.0 |

A variety of stability indicating methods were used to monitor the different physical and chemical properties of the product. These methods were selected from those used for batch release testing of product integrity and included pH, protein concentration, visual appearance, gel permeation chromatography (GP HPLC), SDS PAGE (reducing and non-reducing) and isoelectric focusing. The stability of the product was assessed in each formulation at the intended storage temperature of 5 ± 3°C and the elevated storage temperatures of 25 ± 3°C and 40 ± 3°C. The elevated temperatures were used to provide accelerated stability data in each formulation, which were used to support the selection of formulation determined from the real time data. In addition, samples were stored at - 20 ± 5°C to investigate the effect of freeze-thawing on the stability of the molecule.

To further investigate the effect of the formulation on the tendency of the product to aggregate, samples were stressed by agitation for 48 hours at ambient temperature.

Results generated in this study from each assay indicated candidate formulations where no marked changed occurred at the intended storage temperature or at raised temperature where the changes were least when compared to the study start. Overall, the results of the study suggested that acetate formulations F2, F3, F4 and F5 at pH 5.5 were the most appropriate candidate formulations for the anti-CD3 antibody. Subsequently, 25mM sodium acetate / 125mM sodium chloride / 0.02% Tween 80, pH 5.5 was selected as the final formulation buffer for the anti-CD3 antibody. The formulations provided herein have a pH in the range of 3.0 to 7.0. Preferably, the formulation has a pH in the range of 5.0 to 6.0, and more preferably, the formulation has a pH in the range of 5.2 to 5.8, and most preferably, the formulation has a pH in the range of 5.4 to 5.6. For example, in one embodiment, the formulation has a pH of 5.5.

### EXAMPLE 2: Dosing for Anti-CD3 Antibody Formulations

The dose selection process described herein was used to identify doses that would encompass the therapeutic window for the anti-CD3 antibody formulations to be tested in larger studies. At the same time, the initial dose to be tested was chosen to be non harmful to patients.

As some anti-CD3 monoclonal antibodies described herein, e.g., 28F11, 15C3, 27H5 and 23F10, do not cross-react with "standard" laboratory species CD3, toxicology and efficacy data cannot be obtained in toxicology and preclinical pharmacology. Specifically, GLP studies have demonstrated that there is no cross-reactivity with mouse, rat, rabbit, rhesus monkey, cynomolgus monkey, and baboon CD3 for some of the anti-CD3 monoclonal antibodies described herein. The dose selection process was, therefore, guided by data obtained *in vitro* on human T-cells, comparing an anti-CD3 antibody that does not cross-react with standard laboratory species CD3 (*i.e.,* a "non-cross-reactive CD3 antibody") to other currently marketed anti-CD3 antibodies, using the information published on anti-CD3 antibodies that are currently in development or on the market *(see* e.g., Orthoclone marketed sheet; Woodle Transplantation 1999; Friend Transplantation 1999; Herold NEJM 2002; Keymeulen NEJM 2005):
● Doses clinically used for Orthoclone OKT3, hOKT3γ1 (Ala-Ala) and ChAglyCD3 have been effective at doses between 5 and 25 mg per injection, to achieve 80% or more modulation of CD3 molecules from the surface of T cells and produced a mean serum level of>800ng/mL;
● The length of treatment for Orthoclone OKT3, hOKT3γ1 (Ala-Ala) and ChAglyCD3 ranges between 10-14, 7-14 and 6 consecutive days, respectively;
● *In vitro* studies assessing the modulation of the CD3 and TCR receptors on human T-cells have consistently demonstrated that the IC50 of a non-cross-reactive anti-CD3 antibody is 2-3 fold higher than for Orthoclone;
● *In vitro* studies to assess safety issues such as CRS (cytokine release syndrome) consistently demonstrate that non-cross-reactive anti-CD3 antibodies induce minimal or no TNFa, IFNγ, IL-6 or IL-2 as compared to Orthoclone following incubation with human peripheral blood leukocytes. The parameters of the *in vitro* assay mimic clinical exposure of leukocytes in peripheral blood of patients. The induction of cytokine by a non-cross-reactive anti-CD3 antibody is several orders of magnitude lower than for Orthoclone.

Therefore, to evaluate immunological and preliminary therapeutic efficacy over the therapeutic window, patients will receive 5 daily consecutive doses of a non-cross-reactive CD3 antibody. The dose course, for five consecutive days, in patients will be between 0.5 mg/day and 5.0 mg/day, which is significantly lower than the effective dose of Orthoclone, but will induce a minimal CRS, if any. Preferably, the dose course, for five consecutive days, is between 0.7 mg/day and 2 mg/day. For example, the dose course, for five consecutive days, is 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, or 2.0 mg/day.

The dose courses provided herein result in significantly less severe CRS, based on *in vitro* data.

### EXAMPLE 3: Manufacture and Purification of Anti-CD3 Antibody Formulations

A flow diagram showing the steps in the overall manufacturing process for anti-CD3 antibodies is provided below. Details of the process steps and the in-process controls applied at each stage in the process are presented in tabular format in Tables 2-4.

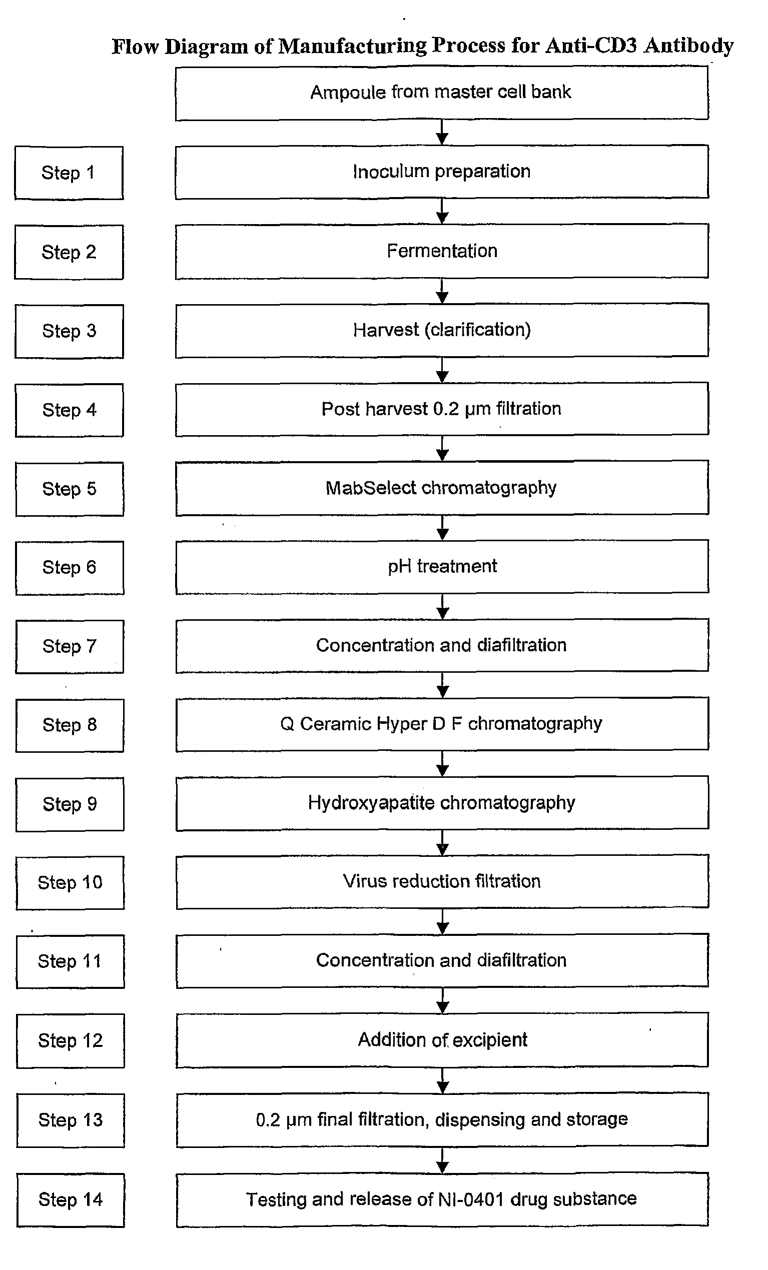

**Table 2: Cell Growth and Harvesting**

| **Step No.** | **Process Step** | **Conditions** | **In Process Controls** | |
|---|---|---|---|---|
| 1 | Inoculum preparation: · Flasks and roller bottles | Growth medium CM41 | Total and viable cell count and % viability Visual inspection for absence of microbial contamination Temperature Roller / shaker rocker speed | |
| | Inoculum preparation: · Cellbags (25 litre) | Growth medium CM41 | Total and viable cell count and % viability Absence of microbial growth Temperature Gas flow Rock angle Rock rate Generation number | |
| 2 | Fermentation: · Production fermenter | Growth medium CM42 | Daily: Total and viable cell count and % viability Absence of microbial growth Temperature pH Dissolved oxygen Nitrogen gas flow rate Nitrogen gas pressure Carbon dioxide gas flow rate Carbon dioxide gas pressure Air gas flow rate Air gas pressure Feed flow rate | |
| 2 | Unprocessed bulk · Production fermenter | | Day of harvest: Bioburden Mycoplasmas: | |
| | | | | ● Hoechst stain |
| | | | | ● Culture |
| | | | Viruses: | |
| | | | | ● *In vitro* viruses (MRC-5,VERO,CHO-K1) |
| | | | | ● Minute virus of mouse by Q-PCR |
| | | | Number of retrovirus like particles/ml of bulk harvest (electron microscopy) | |
| 3 | Harvest/clarification: | Cells and cell debris removed by filtering | | |
| 4 | Post harvest 0.2 µm filtration | Filtration of clarified supernatant into sterile containers. Stored at 5 ± 3°C. Maximum holding time 14 days | Bioburden (pre and post filtration) Endotoxin (post filtration Protein A titre (HPLC) Integrity test of final filters | |

**TABLE 3: PURIFICATION AND MODIFICATION REACTIONS**

| **Step No.** | **Process Step** | **Conditions** | **In Process Controls** |
|---|---|---|---|
| 5 | Purification by mabSelect chromatography | Temp =≥12°C Wash with 50mM Na₃P0₄ /250mM NaCl, pH 7.0 buffer and 50mM Na₃P0₄/1.0M NaCl, pH 7.0 buffer Elute with 10mM sodium formate, pH 3.5 buffer Max load 21 g/l | pH Conductivity Temperature Protein concentration (Protein A HPLC) Bioburden Protein concentration (A₂₈₀) |
| 6 | pH treatment | Adjusted to 3.7 ± 0.10 using 2.0M acetic acid then readjusted to 7.20 ± 0.20 with Tris base | Protein concentration (A₂₈₀) pH Bioburden SDS PAGE¹ |
| 7 | Concentration and Diafiltration | Concentrated to approx. 10.0 ± 1.0 g/l followed by diafiltration into 10mM Na₃P0₄,pH 7.0 buffer | Protein concentration (A₂₈₀) pH Conductivity Bioburden |
| 8 | Purification by Q Ceramic Hyper D F chromatography | Temp ≥12°C **Pre-Equil with 0.1M sodim phosphate, pH 7.0** Wash/Elute with 10mM Na₃P0₄, pH 7.0 buffer Post Elution Wash with 10mM Na₃P0₄/2.0M NaCl, pH 7.0 buffer Max load 50g/l | pH Conductivity Temperature Protein concentration (A₂₈₀) Bioburden SDS PAGE¹ |
| 9 | Purification by hydroxyapatite chromatography | Temp ≥12°C Pre-Equil with 0.1 M sodium phosphate, pH 6.5 Wash with 15mM Na₃P0₄, pH 6.5 buffer Elute with 15mM Na₃P0₄/375mM NaCl, pH 6.5 buffer Max load 20g/l Post Elution wash with 0.5 M Sodium phosphate, pH 6.5 | pH Conductivity Temperature Protein concentration (A₂₈₀) Bioburden SDS PAGE¹ |
| 10 | Planova 20 N virus reduction filtration | 20nm cartridge filter 19 ± 4°C, ≤ 0.98 bar Flush with 15mM Sodium phosphate/375 mM NaCl, pH 6.5 buffer | Protein concentration (A₂₈₀) Temperature Inlet pressure Post use integrity test of filter |
| 11 | Concentration and Diafiltration | Concentrate followed by diafiltration into 25Mm sodium acetate/125mM NaCl, pH 5.5 buffer | Protein concentration (A₂₈₀) pH Conductivity Bioburden |
| 12 | Addition of excipient | 10% polysorbate 80 Final concentration 6.0 ± 0.6 mg/ml. | Protein concentration (A₂₈₀) pH Conductivity |

| | | | |
|---|---|---|---|
| 1- only required if more than 1 cycle is performed and the eluate profiles are not comparable by visual analysis alone | | | |

At the end of each purification processing step and at the time of dispensing into the bulk purified product container, the in-process product is 0.2 µm filtered. For each purification step the time between removing in-process product from 5 ± 3°C storage, performing the purification step and returning processed 0.2 µm filtered product to the cold room must not exceed 36 hours.

The composition of the final formulation buffer is as follows:

| **Buffer Component** | **Concentration** |
|---|---|
| Sodium acetate | 25mM |
| Sodium chloride | 125mM |
| Polysorbate 80 | 0.02% v/v |

**TABLE 4: FINAL FILTRATION AND RELEASE**

| **Step No.** | **Process Step** | **Conditions** | **In process Controls** |
|---|---|---|---|
| 13 | 0.2 µm final filtration and dispensing | Filtration at ambient temperature. Quarantine storage temp 5 ± 3°C | Bioburden (pre filtration) Post use integrity test on filter |
| 14 | Testing and release | Not applicable | According to bulk purified product specification |

### Ram materials used in the manufacturing process for anti-CD3 antibody

CM34 medium, which contains base solutions, is used during cell banking. The initial master cell bank is cryopreserved in CM34 medium supplemented with dimethyl sulphoxide.

CM41 medium, containing base solutions and chemicals, is used during inoculum preparation. Additionally, Supplement E, which contains salts, is added to the basal medium prior to use.

CM42 medium, which contains base solutions, chemicals and amino acids, is used during the fermentation step. CM42 medium is supplemented with SF31, SF32 and Supplement E feeds. These feeds contain base powders, salts, amino acids, glucose, vitamins, chemicals and trace elements. All amino acids used in the media and feed formulations are derived from non-animal sources.

### EXAMPLE 4: Treatment Regimen for Use of Anti-CD3 Antibody Formulations in Treatment of Autoimmune Disease

An anti-CD3 antibody formulation, or matched placebo, is administered by intravenous infusion on study days 1 through 5, at a dose in the range between 0.05 mg/day and 10 mg/day or placebo. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 mg/day, 2.6 mg/day, 2.7 mg/day, 2.8 mg/day, 2.9 mg/day, and 3.0 mg/day.

If a dose level is associated with a significant modulation of the CD3 complex on T-cells and/or with a significant cytokine release syndrome, one or more lower dose level(s) is tested in place of one or more of the higher dose level(s). The desired dose level of the anti-CD3 antibody formulation results in a level of cytokine release that is less than "3" on the WHO scale for cytokine release. The criteria for level 3 on the WHO scale for cytokine release symptoms are shown below in Table 5.

**TABLE 5: WHO TOXICITY GRADING CRITERIA**

| **FLU-LIKE SYMPTOMS (Cytokine release symptoms)** | | | | | |
|---|---|---|---|---|---|
| Fever in absence of (104 0°F) for | none | 37.1 - 38.0°C | 38.1- 40.0°C | > 40.0°C (104.0°F) | > 40.0°C |
| | | | | | |
| infection | | 98.7 -100.4°F | 100.5 -104.0°F | for < 24 hrs | > 24 hrs or with |
| hypotension | | | | | |
| Chills | none | mild or brief | pronounced or prolonged | - - | - - |
| Myalgia/arthralgia | normal | mild | decrease in ability to move | disabled | - - |
| Sweats | normal | mild and occasional | frequent or drenching | - - | - - |
| Malaise | none | mild, able to continue | impaired normal daily activity | in bed or chair > 50% | bed ridden or |
| unable | | | | | |
| | | normal activities | or bedrest <50% of waking hours | of waking hours | to care for self |
| Flu-like symptoms | - - | mild | moderate | severe | life-threatening |
| WEIGHT GAIN | < 5% | 5.0 - 9.9% | 10.0 - 19.9% | ≥ 20% | -- |
| WEIGHT LOSS | < 5% | 5.0 - 9.9% | 10.0 - 19.9% | ≥ 20% | -- |

### EXAMPLE 5: Treatment Regimen for Use of Anti-CD3 Antibody Formulations in Treatment of Transplant Rejection

An anti-CD3 antibody formulation is administered daily until greater than 50% TCR-CD3 coating/saturation is observed. For example, the anti-CD3 antibody formulation is administered daily until the observed TCR-CD3 coating/saturation level is greater than 60%, greater than 70% or greater than 80%. The initial dose is in the range between 0.05 mg/day and 10 mg/day. Preferably, the anti-CD3 antibody formulation is administered in a dosage between 0.1 mg/day to 5.0 mg/day, and more preferably, the anti-CD3 antibody formulation is administered in a dosage between 0.5 mg/day to 3.0 mg/day. For example, the anti-CD3 antibody formulation is administered in a dosage selected from 0.5 mg/day, 0.6 mg/day, 0.7 mg/day, 0.8 mg/day, 0.9 mg/day, 1.0 mg/day, 1.1 mg/day, 1.2 mg/day, 1.3 mg/day, 1.4 mg/day, 1.5 mg/day, 1.6 mg/day, 1.7 mg/day, 1.8 mg/day, 1.9 mg/day, 2.0 mg/day, 2.1 mg/day, 2.2 mg/day, 2.3 mg/day, 2.4 mg/day, 2.5 5 mg/day, 2.6 mg/day, 2.7 mg/day, 2.8 mg/day, 2.9 mg/day, and 3.0 mg/day. If TCR-CD3 saturation is less than 50%, the dose is increased each day until the coating/saturation target is reached. Once the target has been reached, the dosing is followed for 5 days but for a maximum total course of treatment not to exceed 8 days.

The anti-CD3 antibody formulation is administered via intravenous (iv) infusion. Preferably, the iv infusion is continuous infusion over a time frame between 30 minutes and 3 hours, and more preferably between 1 hour and 2 hours. For example, the anti-CD3 antibody formulation is administered via continuous iv infusion for 2 hours each day. Those of ordinary skill in the art will appreciate that the length of time of continuous iv infusion is directly related to the dosage of formulation administered and the volume of the iv bag. Calculating the necessary time for continuous infusion for a given dosage level and bag volume is within the ordinary skill in the art.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A combination of an effective amount of anti-CD3 antibody and an effective amount of an anti-inflammatory or immunosuppressive compound for medical use in a subject.

2. The combination of claim 1 wherein the anti-inflammatory or immunosuppressive compound is selected from the group consisting of methotrexate, cyclosporin A, tacrolimus, corticosteroids, statins, interferon beta, infliximab, etanercept and adalimumab.

3. The combination of claim 1, wherein the anti-CD3 antibody is 28F11, 27H5, 23F10, 15C3, Orthoclone OKT3, human OKT3γ1 (HOKT3γ1) or ChAglyCD3.

4. The combination of claim 1, wherein the effective amount of the anti-CD3 antibody is formulated to provide an amount per dose in the range of 0.05 mg to 10 mg of anti-CD3 antibody.

5. The combination of claim 1, wherein the effective amount of the anti-CD3 antibody is formulated to provide an amount per dose in the range of 0.1 mg to 5.0 mg of anti-CD3 antibody.

6. The combination of claim 1, wherein the effective amount of the anti-CD3 antibody is formulated to provide an amount per dose in the range of 0.5 mg to 3.0 mg of anti-CD3 antibody.

7. The combination of claim 1 wherein the medical use is treatment of an autoimmune disease or inflammatory disorder.

8. The combination of claim 1 wherein the medical use is treatment of rheumatoid arthritis.
